# FASCICULE DE BREVET EUROPEEN

(11) **EP 3 397 751 B1**
(45) Date de publication et mention de la délivrance du brevet: **26.08.2026**
(21) Numéro de dépôt: 16831829.3
(22) Date de dépôt: 29.12.2016
(51) Int. Cl.: C12N 5/071, A61K 35/36, C12Q 1/02, B33Y 80/00

(54) **PROCÉDÉ DE FABRICATION DE SUBSTITUTS CORPORELS PAR DÉPÔT ADDITIF**
VERFAHREN ZUR HERSTELLUNG VON KÖRPERSUBSTITUTEN MITTELS ADDITIVER ABSCHEIDUNG
METHOD FOR MANUFACTURING BODY SUBSTITUTES BY ADDITIVE DEPOSITION

(30) Priorité: 30.12.2015 FR 1563461; 03.03.2016 FR 1651797
(43) Date de publication de la demande: 07.11.2018
(73) Titulaire: Lab Skin Creations, 69004 Lyon (FR); Université Claude Bernard Lyon 1, 69100 Villeurbanne (FR); Centre National de la Recherche Scientifique (CNRS), 75016 Paris (FR); Institut National des Sciences Appliquées de Lyon, 69100 Villeurbanne (FR); Ecole Supérieure de Chimie Physique Electronique de Lyon, 69100 Villeurbanne (FR)
(72) Inventeur: MARQUETTE, Christophe, 69100 Villeurbanne (FR); POURCHET, Léa, 69002 Lyon (FR); THEPOT, Amélie, 69003 Lyon (FR); DOS SANTOS, Morgan, 38160 Saint Verand (FR)
(74) Mandataire: Regimbeau
(86) Numéro de dépôt international: PCT/FR2016/053683
(87) Numéro de publication internationale: WO 2017/115056

(56) Documents cités:
- LI S ET AL: "Direct Fabrication of a Hybrid Cell/Hydrogel Construct by a Double-nozzle Assembling Technology", JOURNAL OF BIOACTIVE AND COMPATIBLE POLYMERS, vol. 24, no. 3, May 2009 (2009-05-01), pages 249 - 265, XP055042727, ISSN: 0883-9115, DOI: 10.1177/0883911509104094
- HUANG Y ET AL: "Rapid prototyping of a hybrid hierarchical polyurethane-cell/hydrogel construct for regenerative medicine", MATERIALS SCIENCE AND ENGINEERING C, vol. 33, no. 6, 6 April 2013 (2013-04-06), pages 3220 - 3229, XP028553342, ISSN: 0928-4931, DOI: 10.1016/J.MSEC.2013.03.048
- ZHAO Y ET AL: "Three-dimensional printing of Hela cells for cervical tumor model in vitro", BIOFABRICATION, vol. 6, no. 3, 035001, 11 April 2014 (2014-04-11), XP055359498, ISSN: 1758-5082, DOI: 10.1088/1758-5082/6/3/035001
- LEE V ET AL: "Design and Fabrication of Human Skin by Three-Dimensional Bioprinting", TISSUE ENGINEERING C, vol. 20, no. 6, June 2014 (2014-06-01), pages 473 - 484, XP055359500, ISSN: 1937-3384, DOI: 10.1089/ten.tec.2013.0335
- DAI X ET AL: "3D bioprinted glioma stem cells for brain tumor model and applications of drug susceptibility", BIOFABRICATION, vol. 8, no. 4, 045005, 11 October 2016 (2016-10-11), pages 1 - 11, XP055359495, DOI: 10.1088/1758-5090/8/4/045005

## Description

### DOMAINE TECHNIQUE

L'invention concerne le domaine de la biotechnologie, et plus particulièrement celui des substituts de tissu corporel, notamment aux substituts de tissu cutanés. Elle concerne notamment la fabrication de substituts corporels (tels que cutanés) destinés à l'implantation dans le corps ou à l'exécution d'essais de principes actifs pharmaceutiques ou cosmétiques ou à l'exécution d'essais de produits chimiques divers, pour évaluer leur toxicité, leur efficacité ou leur pénétration dans le tissu corporel.

### ETAT DE LA TECHNIQUE

En Europe les produits chimiques à visée cosmétique ne doivent plus être testés sur des animaux de laboratoire ; on cherche donc constamment à améliorer les substituts cutanés, à leur donner des caractéristiques plus proches de celles d'un derme naturel, à diminuer leur coût de production et d'utilisation. Parmi les différentes méthodes expérimentales pour fabriquer des matériaux et matrices biotechnologiques ce sont les méthodes de dépôt additif qui ont reçu beaucoup d'attention ces dernières années.

Initialement conçues pour la réalisation de modèles de pièces industrielles en matière plastique (prototypage rapide, voir FR 2 567 668), les méthodes de fabrication additives, dont certaines sont connues sous les termes « stéréolithographie » ou « impression 3D », sont depuis une dizaine d'années explorées dans de nombreux domaines d'application. Elles comprennent en règle générale le dépôt d'une phase poudreuse, pâteuse ou liquide (appelée « encre ») sous une forme tridimensionnelle contrôlée sur un substrat initial, suivie de la solidification de cette phase déposée, de manière à obtenir un objet de forme tridimensionnelle contrôlée. Ledit dépôt se fait le plus souvent en plusieurs passes, conduisant chacune au dépôt d'une phase poudreuse, pâteuse ou liquide de forme tridimensionnelle contrôlée ; ces dépôts sont typiquement solidifiés passe par passe (strate par strate). Ladite phase poudreuse, pâteuse ou liquide peut être homogène (par exemple une poudre homogène ou un fil de polymère thermoplastique en fusion) ou peut comprendre une dispersion de particules solides dans une phase liquide. Sa viscosité peut être un paramètre critique, car la préforme obtenue après son dépôt ne doit pas s'affaisser en attente de sa solidification (cette attente pouvant être d'une fraction de seconde). Sa solidification peut être réalisée par des techniques très différentes selon la composition de l'encre ; elle peut aussi avoir lieu spontanément, notamment dans le cas d'un polymère en extrudé à l'état fondu qui se solidifie, ou dans le cas de pâtes thixotropiques, ou encore dans le cas d'un polymère dont la composition comporte des molécules qui réagissent entre elles (comme cela est décrit dans US 6,942,830).

A titre d'exemple, on peut fabriquer des pièces en matière plastique en déposant une encre polymérisable par la lumière (ou par la chaleur générée par l'absorption) d'un faisceau laser, et on peut fabriquer des pièces métalliques en déposant des pâtes chargées de particules métalliques ou des poudres qui sont consolidées (le plus souvent par fusion intermédiaire) sous l'effet d'un faisceau laser (technique connue sous le sigle SLS, « Selective Laser Sintering ») ; on peut également déposer directement des métaux en fusion. Ainsi on peut fabriquer des pièces industrielles de forme complexe. Une multiplicité de techniques (et de sigles s'y référant) existe sur le marché, telles que le dépôt fil tendu (FDM^{™}, « Fused Deposition Modeling »), le modelage à jets multiples, le FTI (Film Transfer Imaging). Le sigle SFF (Solid Freeform Fabrication ») est utilisé pour désigner un ensemble de techniques capables de fabriquer des structures tridimensionnelles directement à partir de fichiers de CAS (Computer-Aided Design) par des techniques additives.

L'état de la technique décrit la mise en oeuvre des techniques de fabrication additives dans le domaine de la biotechnologie ; on a ainsi imprimé des structures tridimensionnelles à base de matériaux dits biologiques. Plus précisément, il s'agit dans ce cas d'encres à base de matériaux biocompatibles susceptibles d'être solidifiés. Ces matériaux peuvent servir comme support (« scaffold » en anglais) pour des cellules vivantes qui y sont introduites après la solidification du support. On peut également introduire dans les cellules vivantes directement dans l'encre, qui est alors une suspension comportant des cellules vivantes ; ces encres sont parfois appelées « bio-encres ».

Cette solidification peut se faire par voie photochimique. A titre d'exemple, le document US 2014/0052285 décrit l'utilisation de plusieurs types d'hydrogels photopolymérisables, à savoir PED-DA (= diacrylate de polyethylène glycol), PEG-DA-PEA (poly-ester amide), GMA-chitosane et alginate, pour réaliser des supports susceptibles d'être colonisés par des cellules vivantes. US 2013/0304233 décrit la fabrication de structures de support résorbables à base de PPF (fumarate de polypropylène). US 7 780 897 et US 8 197 743 décrivent d'autres modes de mise en œuvre de la stéréolithographie utilisant des hydrogels de polyéthylène glycol solidifiés par une réaction photochimique. La solidification par voie photochimique nécessite cependant des photocatalyseurs, photoinitiateurs et/ou colorants, en quantités significatives (par exemple : 2% de photoinitiateur) ; ces additifs peuvent poser un problème toxicologique. En ce qui concerne le photocatalyseur, même si ce dernier n'est pas toxique en tant que tel, la production de radicaux libre favorise toujours la mortalité cellulaire, et risque de perturber le produit obtenu. Pour cette raison on cherche une alternative à la photopolymérisation pour des applications biotechnologique.

Des hydrogels ont également été utilisés comme milieu pâteux solidifiable ; US 7 051 654 en décrit une vaste liste. US 2014/003932 décrit des hydrogels photoréticulables à base de méthyacrylate. US 2014/0012407 décrit plus spécifiquement des hydrogels thermoréversibles. EP 2 670 669 A1 décrit un procédé dans lequel on dépose une suspension de cellules vivantes comprenant un premier précurseur d'hydrogel capable de se solidifier lorsque la température baisse en-dessous d'une température critique de gélification, et un second précurseur d'hydrogel réticulable, on solidifie la suspension déposée en la refroidissant, et on ajoute un agent réticulant. Le second précurseur peut être un alginate, l'acide hyaluronique, un dérivé de cellulose, du chitosane, de la xanthane, de la fibrine, un gel de pectine, de l'alcool polyvinyle. D'une manière générale, les hydrogels solidifiés pour support de culture cellulaire sont connus de l'homme du métier sous le terme « lattice ». D'autres systèmes solidifiables sont décrits dans US 2013/0164339 et US 2014/00998709.

On connaît également des encres à base d'alginate qui sont solidifiées avec une solution pour fabriquer des supports pour culture cellulaire ; de tels systèmes et procédés sont décrits dans US 8 639 484, US 8 691 974 et US 8 691 274.

US 2013/0017564 décrit le dépôt d'hydroxyapatite pour faire des structures tridimensionnelles sur lesquelles peuvent se fixer des cellules osseuses, les ostéoblastes.

L'impression 3D a aussi été utilisée en biotechnologie pour appliquer une encre comportant des cellules vivantes sur un support favorisant le développement de cultures cellulaires. US 2009/0208466 et WO 2014/039427 décrivent l'impression 3D avec des encres à base de collagène (pouvant comporter de l'alginate) comportant de cellules humaines. US 2011/0250688 décrit le dépôt de cellules musculaires lisses suspendues dans un hydrogel par impression 3D sur un substrat biologique ou synthétique.

US 2012/0089238 décrit le dépôt sur un substrat poreux (scaffold) de quatre types de cellules différentes par impression 3D, chacune déposée en suspension à partir d'une tête d'impression dédiée, pour créer une structure tridimensionnelle complexe mimant un tissu. WO 2013/040087 décrit la fabrication de structures biocompatibles de type « lattice » fabriquées par impression 3D sur lesquels on fixe des cellules vivantes caractéristiques d'une certaine gamme de tissus : cellules musculaires lisses, fibroblastes dermiques, cellules endothéliales, cellules étoilées (en anglais : hepatic stellate cells), hépathocytes, monocytes, macrophages. La publication « Multi-layered culture of human skin fibroblasts and keratinocytes through three-dimensional freeform fabrication » par W. Lee et al., parue dans la revue Biomaterials 30 (2009), p. 1587-1595, décrit le dépôt par impression 3D de cellules sur une lattice d'hydrogel à base de collagène de type I, déposée de la même manière. Cette publication décrit notamment la fabrication de structures multi-couches de collagène dans lesquelles certaines couches incorporent des fibroblastes ou des kératinocytes ; après incubation des cellules elles peuvent servir comme modèle de peau.

La publication *«* On-Demand Three-Dimensional Freeform Fabrication of Multi-Layered Hydrigel Scaffolf With Fluidic Channels" par W. Lee et al., parue en 2010 dans la revue Bitechnology and Bioengineering, vol. 105 (6), p. 1178 - 1186, décrit un procédé de dépôt d'un biomatériau à partir de couches alternées d'un hydrogel à base de collagène et d'une solution de gélatine ; l'hydrogel est réticulé avec du bicarbonate de soude, alors que la solution de gélatine se solidifie par refroidissement lors du dépôt. Les cellules à cultiver sont contenues dans l'hydrogel de collagène. Lors de l'incubation la solution de gélatine devient liquide et est enlevée par rinçage ; ainsi on crée une structure comprenant des couches denses d'hydrogel de collagène qui alternent avec des couches peu denses laissées par le départ de la gélatine. Si ce biomatériau permet la culture de cellules, il ne s'agit cependant pas d'un substitut cutané.

La publication *"*Design and Fabrication of Human Skin by Three-Dimensional Bioprinting" par V. Lee et al., parue dans la revue Tissue Engineering Part C, 20(6) (2014), p. 473-484, montre que les systèmes multicouches obtenues par assemblage manuel et par impressions 3D évoluent de manière totalement différente dans des conditions de culture cellulaire : les systèmes créés par impression 3D se développent bien et gardent leur forme, alors que les systèmes assemblés manuellement se rétrécissent comme une peau de chagrin.

A l'heure actuelle la grande majorité des réalisations d'impression 3D en biotechnologie conduit à des produits pouvant servir comme modèles pour des études scientifiques ; une grande variété de cellules et organes peut être mimée de cette manière. Ces systèmes modèle peuvent permettre de caractériser des principes actifs pharmaceutiques sur des cultures cellulaires élevées et maintenues dans des conditions proches des conditions intracorporelles. La publication *"*Three-dimensional printing of Hela cells for cervical tumor model in vitro" par Y. Zhao et al., parue dans la revue Biofabrication 6 (1914), doi:10.1088/1758-5082/6/3/035001, en fournit un exemple.

A l'opposé, et toujours dans le domaine de la peau humaine, les enjeux thérapeutiques sont énormes car de nombreux besoins insatisfaits existent. Ainsi, le document US 2014/0012225 décrit un dispositif qui permet de déposer sur des zones étendues de la surface du corps humain des hydrogels solidifiables comportant des cellules épidermiques en guise de peau artificielle ; ce dispositif a été imaginé pour traiter des grands brûlés.

La présente invention concerne le domaine des substituts corporels, et notamment des substituts cutanés, destinés soit à être implantés dans le corps, soit à l'exécution d'essais de principes actifs pharmaceutiques ou cosmétiques. Les inventeurs ont constatés que les procédés traditionnels permettant de fabriquer des substituts corporels, et notamment cutanés, prennent beaucoup de temps, sous deux points de vue : tout d'abord ils prennent beaucoup de temps de manipulation (exprimé en heures-homme), car la fabrication des substituts corporels, et notamment cutanés, est un procédé complexe ; puis, le substrat poreux (scaffold) nécessaire à la fabrication des substituts cutanés ne peut pas être utilisé tel quel mais doit d'abord vieillir pendant plusieurs mois (typiquement environ six mois) avant de pouvoir être mis en condition de culture. La phase de culture proprement dite prend au moins six à sept semaines. On note que la manipulation complexe lors de la fabrication implique un risque de contamination des cultures.

Les techniques de fabrication de substituts cutanés à partir d'une bio-encre peuvent être automatisées facilement, elles présentent une bonne reproductibilité et permettent ainsi d'obtenir un produit normalisé et sont plus rapides : le biomatériau est mélangé avec les cellules le jour de la production pour former la bio-encre, et la maturation du derme se fait en dix à quinze jours.

Cependant, le résultat n'est pas très bon, car on n'obtient pas de derme artificiel permettant la culture d'un épiderme stratifié à sa surface, qui puisse servir comme modèle réaliste d'une peau naturelle. En effet, la publication « Standardized 3D Bioprinting of Soft Tissue Models with Human Primary Cells » par M. Riemann et al., parue en 2015 dans J. of Laboratory Automation, p. 1 - 14 (doi : 10.1177/2211068214567146) décrit la fabrication d'un substitut cutané par dépôt additif de couches successives (épaisseur individuelle : 0,05 mm) d'une bio-encre photopolymérisable ; une couche de matrice est alternée avec une couche comprenant des fibroblastes. Chaque couche est immédiatement photopolymérisée. La bio-encre est à base de PEG. Le substitut cutané ainsi obtenu ne présente cependant pas la structure stratifiée d'un épiderme naturel, et il comprend de nombreux trous.

Le problème de fabriquer, en faisant intervenir des techniques additives, un substitut cutané qui présente une ressemblance structurelle et fonctionnelle suffisante avec la peau naturelle reste sans solution.

### Objet de l'invention

Selon l'invention le problème a été résolu par l'utilisation d'une composition de bio-encre nouvelle, comprenant un mélange de gélatine naturelle, d'alginate, et de fibrinogène qui est solidifiée par trois moyens différents : lors du dépôt de l'encre, la gélatine solidifie par refroidissement, ce qui permet de garder la forme du boudin d'encre déposé. Après dépôt on traite l'objet déposé par avec une solution comportant des ions de calcium et de la thrombine, afin de solidifier l'alginate par les ions de calcium et de coaguler le fibrinogène par l'effet de la thrombine.

Ainsi, un premier objet de la divulgation est un procédé de fabrication d'une bio-encre pour dépôt additif, dans lequel :
(a) On approvisionne une première solution comprenant entre 5% et 40% massiques de gélatine (de préférence entre 6% et 30% massiques) et entre 0 % et 5% massiques de NaCl ;
(b) On approvisionne une seconde solution comprenant entre 1% et 12% massiques d'alginate (de préférence entre 1% et 8% massiques) et entre 0% et 5% de NaCl ;
(c) On approvisionne une troisième solution comprenant entre 1% et 15% massiques de fibrinogène (de préférence entre 3% et 15% massiques), et optionnellement des cellules vivantes (telles que des fibroblastes) en suspension ;
(d) On crée un mélange comprenant :
   environ 35% à 65% volumiques de la première solution ;
   environ 15% à 35% volumiques de la seconde solution ;
   environ 15% à 35% volumiques de la troisième solution ;
   ces proportions étant choisies de manière à s'additionner à 100%,
et dans lequel procédé :
- l'ordre des étapes (a),(b) et (c) est indifférent,
- la teneur en NaCl est choisie de manière à ce que dans ladite première solution et ladite seconde solution réunies elle soit comprise entre 0,2% et 5% massiques, de préférence entre 0,2 et 3% massiques, et encore plus préférentiellement entre 0,4% et 2% massiques.

Pour la préparation de substituts cutanés, lesdites cellules vivantes peuvent dériver des différentes structures de la peau : derme, épiderme, hypoderme, vaisseaux sanguins et lymphatiques, follicule pileux, glandes sébacées, glandes sudoripares, pores, muscles érecteur du poil, muscles, corpuscule de Meissner, corpuscule de Pacini, corpuscule de Ruffini, tissu conjonctif, membrane basale. Plus particulièrement ces cellules peuvent être des kératinocytes, mélanocytes (notamment des phototypes I, II, III, IV et V selon la classification de Fitzpatrick), fibroblastes (incluant les fibroblastes papillaires, réticulaires), cellules de Merkel, cellules de Langerhans, sébocytes, cellules dendritiques dermiques, macrophages, mastocytes, cellules épithéliales des follicules pileux, fibroblastes de la papille du follicule pileux, préadipocytes, cellules souches (notamment celles du tissu adipeux), neurones sensitifs, cellules musculaires. Ces cellules peuvent être saines ou pathologiques.

Un autre objet de la présente divulgation est la bio-encre susceptible d'être obtenue à partir de ce procédé. Elle peut contenir des cellules vivantes, et notamment des fibroblastes. La gélatine confère à cette bio-encre une viscosité qui présente un point de transition (point de gélification) à une température se situant typiquement entre 27°C et 32°C, et de préférence entre 28°C et 30°C : la bio-encre est fluide au-dessus de cette température et gélifie au-dessous de cette température. Cette gélification a notamment lieu lorsque la bio-encre, portée à une température T1 supérieure à son point de gélification, est déposée sur un substrat se trouvant à une température T2 inférieure à ce point de gélification : dans ce cas elle peut se solidifier immédiatement sans s'étaler complètement, éventuellement en gardant la forme du boudin d'extrusion. Ainsi, la bio-encre peut être utilisée dans des procédés de dépôt additif.

L'invention concerne un procédé de fabrication d'un substitut de tissu corporel, dans lequel :
(i) on approvisionne une bio-encre;
(ii) on approvisionne une solution aqueuse (dite « solution de polymérisation ») comprenant entre 1% à 5% massiques d'ions de calcium et entre 2 U/mL et 40 U/mL (et de préférence entre 5 U/mL et 40 U/mL, et encore plus préférentiellement entre 10 U/mL et 30 U/mL) de thrombine ;
(iii) on porte ladite bio-encre à une température T1 supérieure à son point de gélification et on la dépose sur un substrat se trouvant à une température T2 inférieure au point de gélification de ladite bio-encre, où elle gélifie pour former un objet de forme tridimensionnelle contrôlée dit « objet imprimé brut »,
(iv) on traite ledit objet imprimé brut avec ladite solution de polymérisation pour consolider ledit objet imprimé brut en un substitut de tissu corporel ;
(v) optionnellement, dans le cas où ladite bio-encre comporte des cellules vivantes, on incube ledit précurseur de substitut corporel dans un milieu de culture cellulaire. Cette incubation est avantageusement effectuée à une température comprise entre 36°C et 38°C, de préférence dans une atmosphère humide avec 5% de CO₂.

Dans ce procédé de fabrication d'un substitut de tissu corporel, la bio-encore approvisionnée à l'étape (i) est obtenue par un procédé de fabrication dans lequel :
(a) On approvisionne une première solution aqueuse comprenant entre 5% et 40% massiques de gélatine, de préférence entre 6% et 30% massiques, et entre 0 % et 5% massiques de NaCl ;
(b) On approvisionne une seconde solution aqueuse comprenant entre 1% et 12% massiques d'alginate, de préférence entre 1% et 8% massiques, et entre 0% et 5% de NaCl ;
(c) On approvisionne une troisième solution aqueuse comprenant entre 1% et 15% massiques de fibrinogène, de préférence entre 3% et 15% massiques, et des cellules vivantes en suspension ;
(d) On créé un mélange comprenant :
   environ 35% à 65% volumiques de la première solution ;
   environ 15% à 35% volumiques de la seconde solution ;
   environ 15% à 35% volumiques de la troisième solution ;
   ces proportions étant choisies de manière à s'additionner à 100%,
et dans lequel:
- l'ordre des étapes (a),(b) et (c) est indifférent,
- la teneur en NaCl est choisie de manière à ce que dans ladite première solution et ladite seconde solution réunies elle soit comprise entre 0,2% et 5% massiques, de préférence entre 0,2 et 3% massiques, et encore plus préférentiellement entre 0,4% et 2% massiques,
- lesdites cellules vivantes sont des cellules primaires isolées de tout tissu ou organe humain ou animal ou des cellules souches animales ou des cellules souches humaines non embryonnaires.

Ce procédé peut être réalisé par dépôt simple ou par dépôt additif, le dépôt simple comprenant le dépôt d'une seule couche ou d'un seul boudin d'encre, le dépôt additif permettant la création d'objets imprimés bruts ayant une certaine extension bidimensionnelle contrôlée, voire même d'objets ayant une forme tridimensionnelle contrôlée. Notamment, ce procédé peut comprendre le dépôt (simple ou additif) par extrusion (par exemple à partir d'une seringue pourvue d'un piston ou d'une vis), par jet d'encre (impliquant le plus souvent la projection de gouttelettes de bio-encre sur un substrat se trouvant à la température appropriée pour permettre la solidification de la bio-encre) ou par laser (impliquant par exemple le dépôt d'une couche de bio-encre sur une couche formée d'un matériau capable d'absorber la lumière du laser, cette dernière couche étant ensuite irradiée de manière localisée par le laser, ce qui engendre la projection de gouttelettes de l'encre sur un substrat tenu à la température appropriée pour solidifier la bio-encre).

De manière avantageuse T1 est compris entre 28°C et 37°C (de préférence entre 28°C et 33°C) et T2 est compris entre 0°C et 20°C (et de préférence entre 4°C et 18°C).

C'est le traitement de l'objet imprimé brut avec la solution de polymérisation qui le consolide définitivement : si la coagulation de l'alginate par le calcium peut être au moins partiellement réversible par le départ éventuel du calcium, la coagulation du fibrinogène par la thrombine est irréversible. Dans le procédé selon l'invention cette consolidation est homogène dans l'épaisseur de l'objet.

Le traitement dudit objet imprimé brute avec ladite solution de polymérisation peut être effectué par trempage, de préférence à une température T3 supérieure à T1 et de préférence comprise entre 35°C et 38°C.

Selon un mode de réalisation particulier de l'invention, ledit précurseur de tissu corporel est un précurseur de substitut cutané. Dans ce cas, ladite bio-encre comprend des cellules vivantes en suspension qui sont des fibroblastes. Ladite incubation est effectuée à une température comprise entre 36°C et 38°C, en atmosphère humide et sous 5% CO₂. Elle comprend avantageusement une première phase d'incubation comprise entre un et quarante jours, et une deuxième phase d'incubation entre cinq et quarante jours, sachant qu'entre la première et la deuxième phase on dépose sur la surface dudit substitut cutané une suspension aqueuse de kératinocytes. On peut également déposer les kératinocytes à la surface dudit substitut cutané par dépôt d'une bio-encre utilisée dans le procédé selon l'invention, notamment en une couche mince.

Ledit précurseur de substitut cutané peut avoir une forme plate ou autre. Dans un mode de réalisation ledit objet imprimé brut comprend une surface supérieure sensiblement plate et présente une répartition homogène de fibroblastes, qui est comprise entre 0,2 et 10 x 10⁵ (et de préférence entre 0,2 et 2 x 10⁵) fibroblastes par cm² de surface supérieure plate.

Dans un autre mode de réalisation la concentration de fibroblastes est comprise entre 0,6 et 12 x 10⁵ fibroblastes par cm³ de bio-encre, et de préférence entre 1 et 7 x 10⁵ fibroblastes par cm³ de bio-encre, et encore plus préférentiellement entre 1 et 5 x 10⁵ fibroblastes par cm³ de bio-encre ; ce mode de réalisation est préféré.

Toujours dans le but de préparer un substitut cutané, la quantité de kératinocytes déposés est avantageusement comprise entre 0,05 et 50 x 10⁵ (et de préférence entre 0,2 et 20 x 10⁵ kératinocytes par cm² de surface supérieure plate ; la concentration la plus préférée se situe entre 0,5 et 10 x 10⁵ kératinocytes par cm² de surface supérieure plate.

Le procédé selon l'invention permet la fabrication de substituts corporels les plus divers. Il permet notamment la fabrication de substituts cutanés comprenant une couche de derme comprenant notamment des fibroblastes, une couche d'épiderme, et une couche cornée comprenant notamment des kératinocytes et des mélanocytes. Cette structure est bien stratifiée et différenciée. Le substitut cutané préparé par le procédé selon l'invention peut être utilisé pour étudier l'effet de principes actifs cosmétiques ou dermatologiques, ou encore pour des études toxicologiques. Il peut également être destiné à être utilisé en chirurgie réparatrice, esthétique ou plastique.

A titre d'exemple, les substituts corporels réalisés avec des cellules pathologiques peuvent être utilisés pour tester l'efficacité générale ou individuelle de principes actifs.

### Description des figures

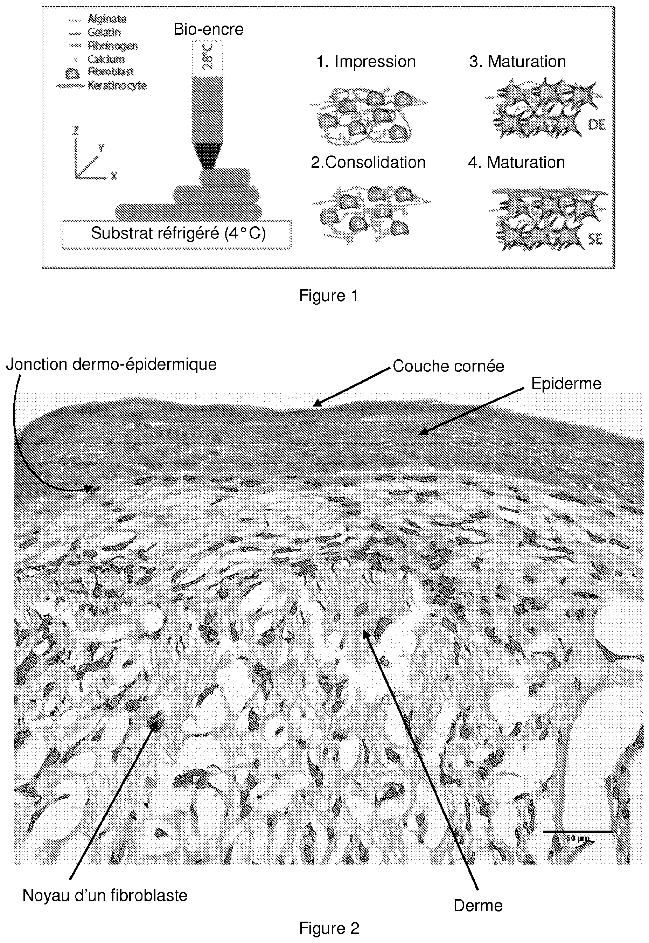

Les figures 1 à 17 illustrent certains aspects de l'invention, mais ne limitent pas la portée de l'invention.

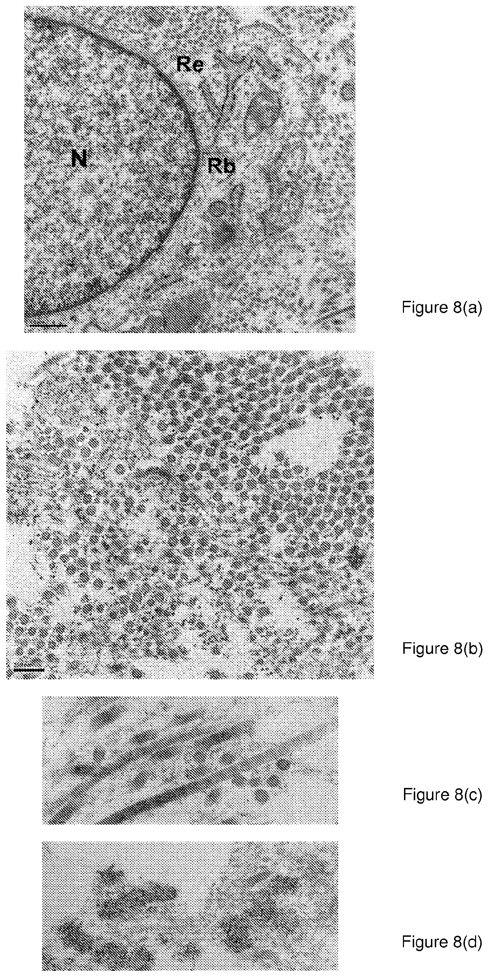

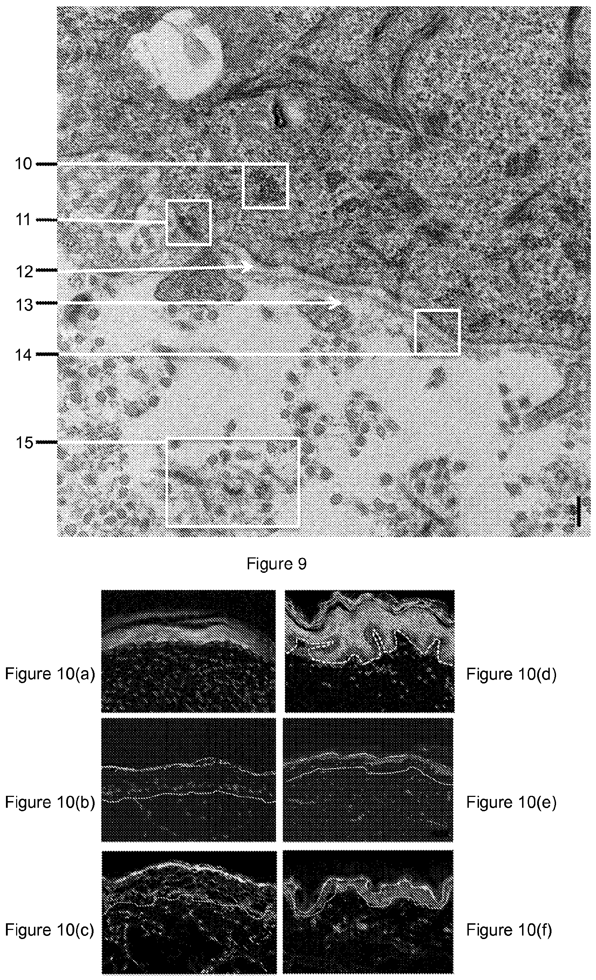

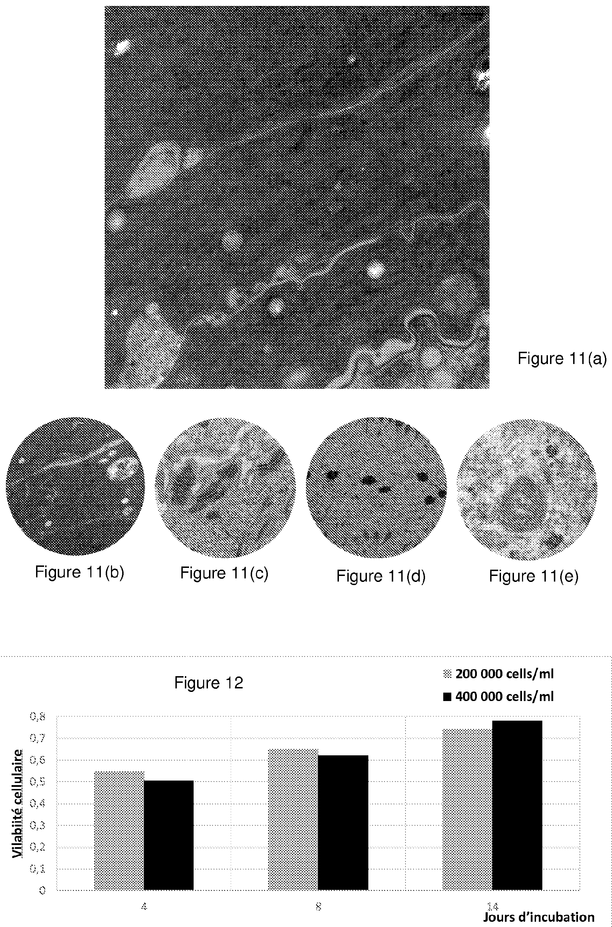

Les analyses histologiques et immunohistologiques sont basées sur des techniques connues de l'homme de métier. Les micrographies optiques sont basées sur des coupes tomographiques (5 µm) enrobées dans la paraffine et fixées à la formaline, qui ont été colorées après déparaffinage et réhydratation. Une caméra CCD de type DS-Ri1 couplée à un logiciel de type NIS (société Nikon) a été utilisée pour générer les images en format 16 bits non compressé (six observations représentatives par échantillon). Les expériences d'immunofluorescence ont été effectuées sur des cryosections de 5 µm avec des anticorps appropriés, et les échantillons ont été observés avec un microscope optique Observer Z1 couplé à un système de balayage laser confocal LSM700 (Zeiss) pour générer des images en format 8 bits non compressé (six observations représentatives par échantillon). Les échantillons pour microscopie électronique par transmission ont été fixés avec un tampon (pH 7,5) de cacodylate de sodium (0,1 M) contenant 2% de glutaraldehyde à 4°C, puis traitées avec une solution de OsO₄ à 1%. Les échantillons ont été déshydratés puis enrobés dans une résine époxy qui a été polymérisée pendant 3 jours à 56°C. Les coupes ont ensuite été traitées avec une solution d'acétate d'uranyle (7%) et l'acétate de plomb.
La figure 1 montre de manière schématique le procédé selon l'invention.
La figure 2 se rapporte à l'exemple 2 et montre une micrographie optique d'une coupe transversale à travers un substitut cutané obtenu par le procédé selon l'invention. La barre noire en bas à droite représente la longueur de 50 µm.

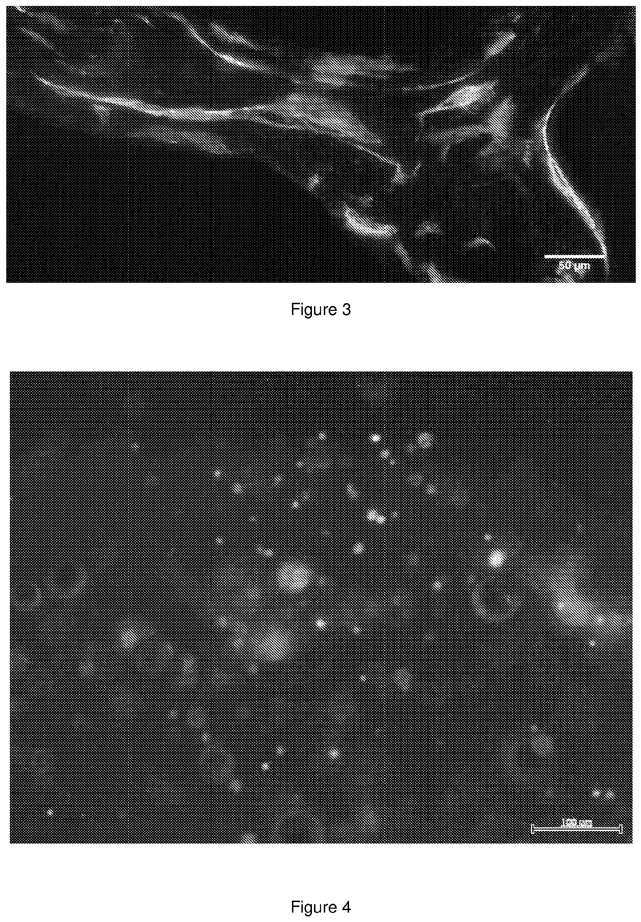

La figure 3 se rapporte à l'exemple 3 et montre une micrographie optique d'une coupe transversale à travers un substitut cutané obtenu par le procédé selon l'invention, avec un marquage immuno-histochimique de la vimentine. La barre blanche en bas à droite représente la longueur de 50 µm.
La figure 4 se rapporte à l'exemple 4b (hors invention) et montre une micrographie optique d'une coupe transversale à travers un substitut cutané réalisé avec une bio-encre hors invention. La bio-encre est dépourvue de fibrinogène et contient des fibroblastes de type NIH3T3 couplés avec la protéine GFP (Green Fluorescent Protein). La figure montre que lorsque la bio-encre ne comporte pas de fibrinogène les fibroblastes ne s'étalent pas en trois dimensions, n'adhèrent pas à la bio-encre solidifiée, et finissent par mourir.

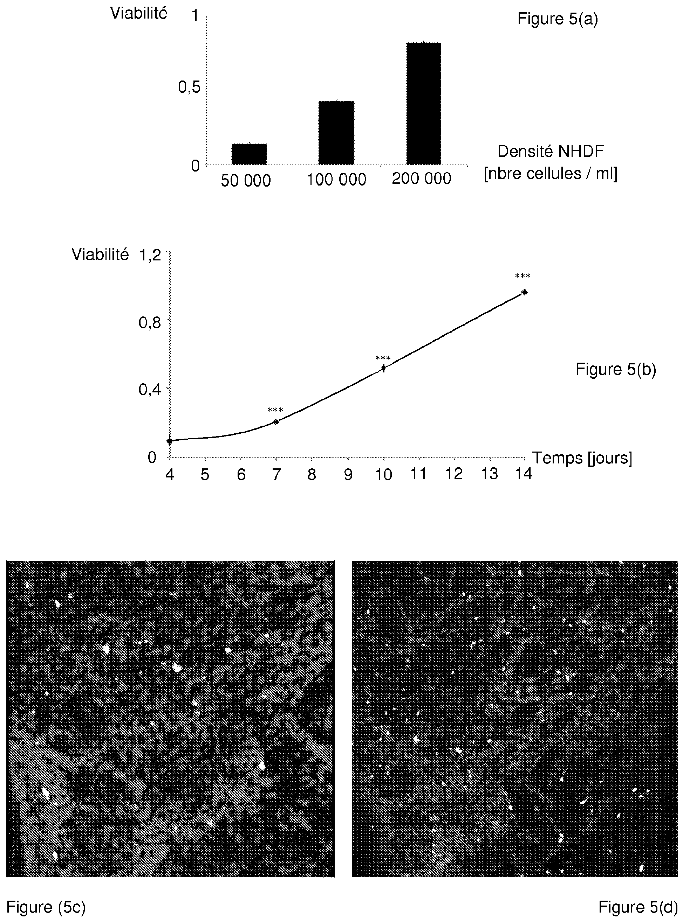

La figure 5 se rapporte à l'exemple 6. La figure 5(a) montre, pour des précurseurs de substitut cutané réalisés avec trois encres comprenant chacune une concentration de cellules vivantes différente, la viabilité cellulaire, exprimée en rapport d'absorbance optique après coloration au bleu Alamar^{™} (mesure de l'absorbance à 570 nm et 600 nm, la quantité de cellules vivantes résulte du rapport entre ces deux valeurs d'absorbance). La figure 5(b) montre la viabilité cellulaire (déterminée de la même manière) après 7, 10 et 14 jours d'incubation pour un substitut cutané réalisée avec une bio-encre chargée de 100 000 fibroblastes / mL ; l'augmentation du nombre de cellules reflète la prolifération cellulaire.
Les figures 5(c) et (d) montrent une coupe en microscopie après coloration au DAPI qui engendre le voile gris représentant les cellules vivantes, et au iodure de propidium qui engendre les taches blanches représentant les cellules mortes, sachant que la figure 5(c) se rapporte au substitut corporel après 14 jours d'incubation, alors que la figure 5(d) se rapporte au même substitut corporel après un traitement au sodium dodécyl sulfate (0,5%) qui tue les cellules (expérience de contrôle).

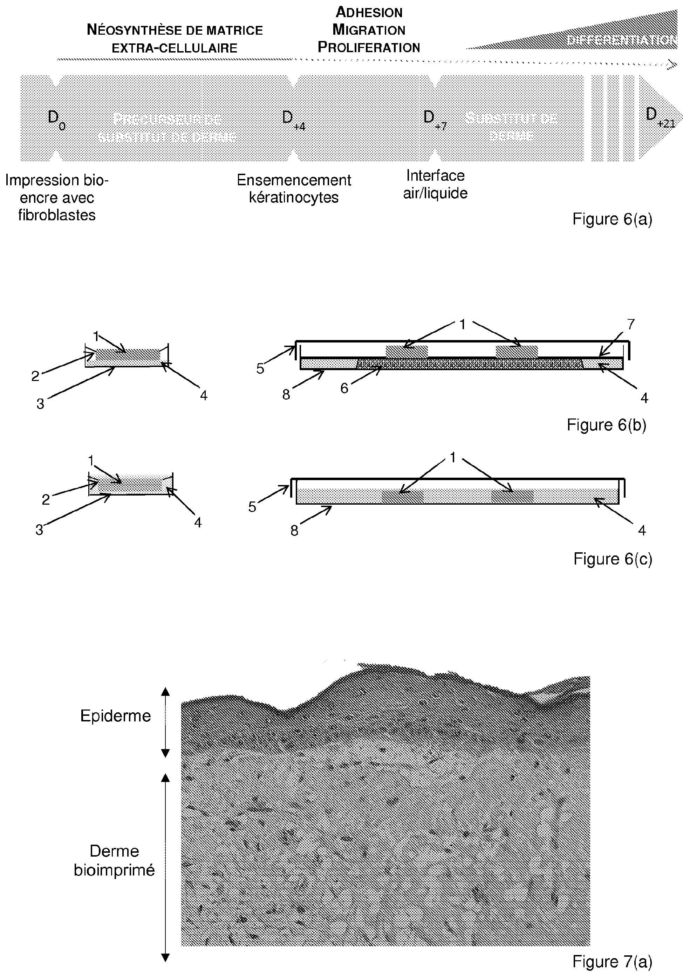

La figure 6(a) illustre de manière schématique le déroulement d'un mode de réalisation du procédé selon l'invention. Les figures 6(b) et 6(c) montrent de manière schématique la position de l'échantillon pendant les premiers sept jours d'incubation en immersion dans le milieu de culture (figure 6(c)) et à la surface du milieu de culture (figure 6(b)) dans le puits d'une plaque multi-puits (à gauche) et dans une boîte de culture (figure 6(c)).

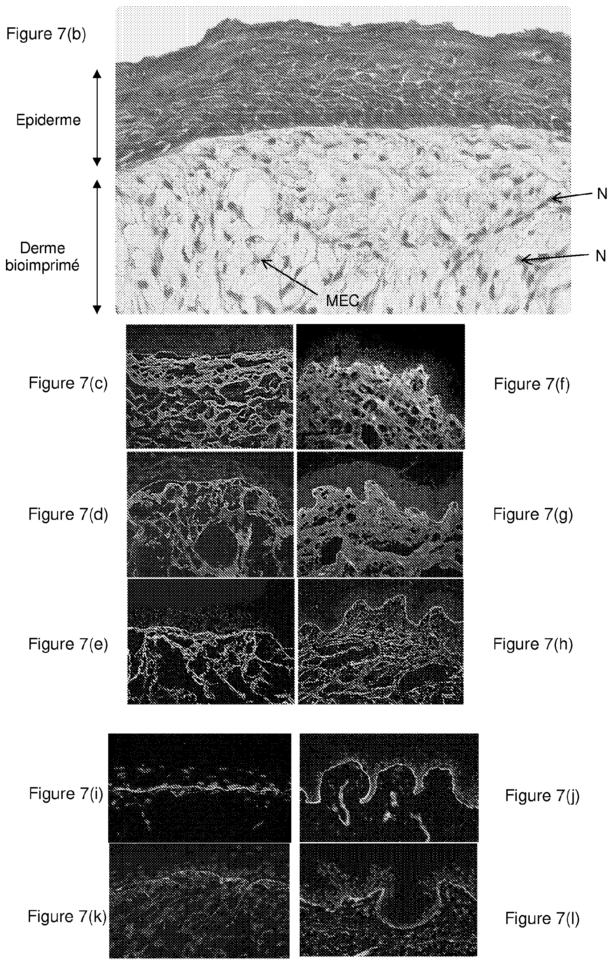

Les figures 7 à 11 se rapportent à l'exemple 7 et montrent :
   - des coupes de microcopie optique à travers un substitut cutané obtenu par le procédé selon l'invention (figure 7(a) avec coloration HPS (hématoxyline - phloxine - safran), figure 7(b) avec coloration au trichrome de Masson) ;
   - Des images de marquage immuno-histologique spécifique (figures 7(c),(e),(f)*,(g)*, (h)*,(i),(j),(k)* et (l)*, figures 10(a),(b),(c),(d)*,(e)*,(f)*, comme expliqué dans l'exemple, sachant que les figures marquées d'un astérisque se rapportent à une
   peau humaine saine, les autres au substitut de derme obtenus par le procédé selon l'invention ;
- Des images de microscopie électrique à transmission (figures 8(a),(b),c),(d), figures 11(a),(b),(c),(d),(e)) d'un substitut de derme obtenu par le procédé selon l'invention.

Les figures 12, 13, 14, et 15 se rapportent, respectivement, aux exemples 8, 9, 10 et 11 et montrent des courbes de prolifération cellulaire après incubation de substituts corporels obtenus par le procédé selon l'invention.

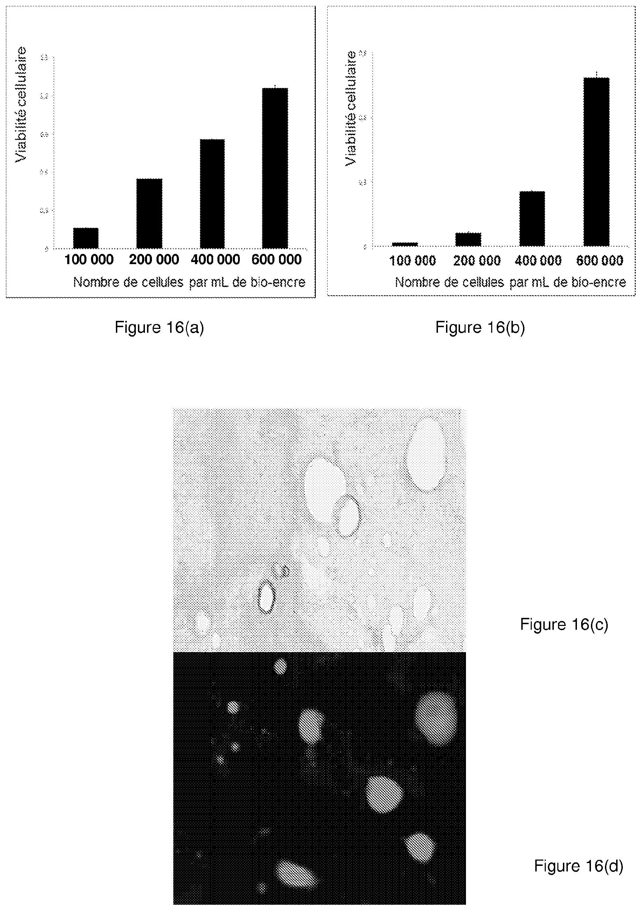

La figure 16 se rapporte à l'exemple 12 et montre des courbes de viabilité cellulaire pour deux bio-encres utilisées dans le procédé selon l'invention différentes (figures 16(a),(b)), chacune préparée avec 4 concentrations différentes, et des micrographies (figures 16(c) et (d)). L'une est préparée avec des cellules souches du tissu adipeux (figure 16(a)) et l'autre avec des préadipocytes (figure 16(b)), deux types de précurseurs des adipocytes. Lorsque ces substituts d'hypoderme sont bio-imprimés avec le procédé selon l'invention puis différenciés avec des facteurs de différenciation (connus de l'homme du métier), un hypoderme fonctionnel est obtenu, puisque les cellules présentent des vacuoles lipidiques remplies d'acides gras typiques des adipocytes. La micrographie de la figure 16(c) montre un immuno-marquage de la périlipine A, une protéine typiquement exprimée à la surface des gouttelettes lipidiques. La micrographie de la figure 16(d) montre une coloration du contenu lipidique des cellules, cette coloration ayant été obtenue au rouge de Nile, un colorant spécifique des lipides.

La figure 17 se rapporte à l'exemple 13 en montre des photographies d'un précurseur de substitut corporel réalisé par le procédé selon l'invention à différents stades de fabrication.

### Description détaillée

La présente invention permet de créer des substituts corporels, et notamment des dermes équivalents, par le biais du dépôt additif (tel que l'impression 3D). Ce nouveau procédé de fabrication permet un gain de temps et une facilité de fabrication des substituts corporels (et notamment de substituts de derme) extrêmement avantageuse, simple et reproductible. Les temps de fabrication et de maturation sont très réduits. Ce procédé de fabrication peut utiliser notamment les techniques de dépôt additif suivantes : le dépôt (simple ou additif) par extrusion, le dépôt par jet d'encre, et le dépôt par laser.

Le procédé de fabrication d'un substitut de tissu corporel selon l'invention est tel que défini dans les revendications 1-9 annexées.

La bio-encre qui est utilisée dans le procédé selon l'invention est composée d'un mélange de biomatériaux susceptibles de former un hydrogel et de cellules. Elle peut être préparée juste avant son emploi à partir de solutions aqueuses stables. Une fois que solutions préparées avec les bonnes concentrations, il suffit de les mélanger délicatement avec une suspension de cellules (si un substitut cutané est visé : typiquement des fibroblastes) dans une seringue pour extrusion. Ensuite, la seringue est montée sur la bio-imprimante. Avantageusement on prévoit un pousse-seringue afin d'avoir un contrôle de débit ; à titre d'exemple le pousse-seringue peut être contrôlé par une vis sans fin et un moteur qui exerce une pression constante sur le liquide ou gel à extruder. Ainsi on imprime un objet en hydrogel ; cet objet est typiquement un objet plat. Une fois l'hydrogel imprimé, il est nécessaire de l'immerger dans une solution de polymérisation biocompatible, ce qui va permettre aux biomatériaux de former un réseau solide et de maintenir la forme tridimensionnelle souhaitée. Ensuite, dans une étape appelée aussi « étape de maturation » on laisse les cellules se développer. Au cours de ce développement cellulaire en réseau tridimensionnel les cellules imprimées (dans le cas d'un substitut cutané : fibroblastes) contenues dans l'hydrogel polymérisé sécrètent leur matrice extracellulaire. L'hydrogel va alors progressivement se résorber pour laisser place à l'émergence d'un tissu néosynthétisé où des fonctions spécifiques vont apparaitre, ce qui est un avantage certain par rapport à une approche de type « scaffold » où les biomatériaux sont quelquefois non-résorbables.

Si un substitut cutané est visé, on ajoute avantageusement, par dépôt d'une bio-encre ou par ensemencement de la surface, des kératinocytes ; cette étape est suivie d'une incubation appropriée et permet ainsi d'obtenir un substitut cutané stratifié et différencié comportant le derme, la jonction dermo-épidermique, l'épiderme et la couche cornée.

Pour la mise en œuvre du procédé selon l'invention, la bio-encre doit être préparée selon des proportions et un ordre prédéfini et précis.

Dans une première étape on approvisionne des poudres d'alginate et de gélatine, on les stérilise et solubilise pour obtenir deux solutions stock : la première est une solution aqueuse de gélatine, obtenue par dissolution de poudre de gélatine (de préférence à une concentration comprise entre 5% m/v et 40% m/v) dans une solution de NaCl (de préférence à une concentration comprise entre 0,2% m/v et 5% m/v, plus préférentiellement entre 0,4% m/v et 3% m/v, et encore plus préférentiellement entre 0,5% m/v et 1,4% m/v), la deuxième est une solution aqueuse d'alginate, obtenue par dissolution de poudre d'alginate (de préférence de qualité dite « Very Low Viscosity ») à une concentration comprise entre 1% m/v et 10% m/v (de préférence entre 2% m/v et 7% m/v) dans une solution de NaCl (de préférence à une concentration comprise entre 0,2% m/v et 5% m/v, plus préférentiellement entre 0,4% m/v et 3% m/v, et encore plus préférentiellement entre 0,5% m/v et 1,4% m/v). Ces deux solutions sont stables et peuvent être stockées.

Avantageusement on utilise la même solution de NaCl pour préparer ces deux solutions. Les gammes de concentration suivantes sont particulièrement avantageuses :
- Solution de gélatine entre 6% m/v et 30% m/v, solution d'alginate entre 1% m/v et 8% m/v ; solution de NaCl entre 0,2% m/v et 3% m/v (de préférence entre 0,4% m/v et 2% m/v, et encore plus préférentiellement entre 0,5% m/v et 1,4% m/v).
- Solution de gélatine entre 10% m/v et 30% m/v, solution d'alginate entre 2% m/v et 6% m/v ; solution de NaCl entre 0,2% m/v et 3% m/v (de préférence entre 0,4% m/v et 2% m/v, et encore plus préférentiellement entre 0,5% m/v et 1,4% m/v).
- Solution de gélatine entre 15% m/v et 26% m/v, solution d'alginate entre 2% m/v et 6% m/v (et de préférence entre 3% m/v et 5% m/v) ; solution de NaCl entre 0,2% m/v et 3% m/v (de préférence entre 0,4% m/v et 2% m/v, et encore plus préférentiellement entre 0,5% m/v et 1,4% m/v).
- Solution de gélatine entre 17% m/v et 24% m/v, solution d'alginate entre 2% m/v et 6% m/v (et de préférence entre 3% m/v et 5% m/v) ; solution de NaCl entre 0,4% m/v et 2% m/v (préférentiellement entre 0,5% m/v et 1,4% m/v, et encore plus préférentiellement entre 0,6% m/v et 1,3% m/v).

Le rôle du NaCl dans ces solutions est de favoriser un environnement osmotique favorable au développement cellulaire dans la bio-encre, et de faciliter la dissolution de l'alginate.

La poudre d'alginate est un produit commercialement disponible, et on préfère la qualité dite « Low Viscosity », et encore plus préférentiellement la qualité dit « Very Low Viscosity ».

Si la bio-encre comporte trop d'alginate elle devient trop dure pour être utilisable pour des techniques de dépôt additif, si elle en contient trop peu sa polymérisation par les ions de calcium est insuffisante et peut ne pas conduire à un précurseur suffisamment solide.

Si la bio-encre comporte trop de fibrinogène elle devient trop dure pour être utilisable pour des techniques de dépôt additif, si elle en contient trop peu les cellules n'adhèrent pas à la bio-encre, ne s'étalent pas et finissent par périr. Pour cette raison, une concentration massique de 0,2 % de fibrinogène dans la bio-encre est nécessaire, et on préfère une teneur minimale de 0,3%. De même, la concentration de thrombine doit être choisie de manière à permettre la transformation du fibrinogène en fibrine.

Par ailleurs, on prépare une troisième solution qui est une solution aqueuse de fibrinogène, de préférence à une concentration comprise entre 1% m/v et 15% m/v (plus préférentiellement entre 3% m/v et 12% m/v, et encore plus préférentiellement entre 5%m/v et 10% m/v) dans laquelle on a introduit une concentration cellulaire appropriée (typiquement entre 0,05 et de 1 millions cellules/mL (de préférence entre 0,1 et 0,6 millions par mL). Pour un essai de dépôt de précurseur de substitut cutané on peut par exemple préparer 2 mL de cette solution. Lesdites cellules sont notamment des fibroblastes.

La concentration cellulaire dans la bio-encre est critique pour la réussite du procédé de préparation de substitut corporel selon l'invention. Si elle trop faible, les cellules ne sont pas capables de former un tissu suffisamment proche du tissu natif visé. A titre d'exemple, dans le cas des fibroblastes il ne se forme pas assez de matrice extracellulaire pour remplacer les constituants de la bio-encre. Dans le cas les cellules épithéliales comme les kératinocytes, on observe dans ce cas que les cellules ne sont pas jointives et finissent par mourir.

Si la concentration cellulaire dans la bio-encre est trop forte la structure se dégrade au lieu de se développer, on observe de manière surprenante qu'elle change de texture et devient inutilisable (elle « fond » littéralement).

Dans une troisième étape on prépare la bio-encre à partir de ces trois solutions de manière à obtenir un mélange qui contient environ 35% à 65% (de préférence environ 45% à 55%, et encore plus préférentiellement environ 50%) de la première solution (gélatine), environ 15% à 35% (de préférence environ 20% à 30%, et encore plus préférentiellement environ 25%) de la deuxième solution (alginate) et environ 15% à 35% (de préférence environ 20% à 30%, et encore plus préférentiellement environ 25%) de la troisième solution pour un total de 100%, ces pourcentages étant exprimés en pourcent volumiques.

Dans une quatrième étape on prépare une quatrième solution aqueuse, qui est la solution de polymérisation. Elle comprend du calcium en solution à raison d'environ 1 à 5% m/v et de préférence environ 3% m/v, à laquelle on ajoute de la thrombine à une concentration finale d'environ 10 U/mL. Cette solution de polymérisation doit être approvisionnée en quantité suffisante pour permettre d'immerger l'objet obtenu par impression 3D afin d'obtenir un gel polymérisé de manière homogène. Cette quatrième étape peut être réalisée avant ou au cours des trois étapes précédentes, mais la durée de stockage de cette quatrième solution est limitée ; les inventeurs ont observé que lorsque l'on prépare la quatrième solution juste avant son emploi, on obtient une solidification et polymérisation plus homogène de la bio-encre.

Si ladite quatrième solution ne comporte pas assez de calcium la polymérisation de l'alginate ne se produit pas ou n'est pas satisfaisante. Si elle comporte trop de calcium cela diminue la viabilité des cellules, le calcium pouvant être cytotoxique.

Dans une cinquième étape on procède à l'impression, c'est-à-dire au dépôt de l'encre sur un support. A cette fin l'encre est portée à une température T1 suffisante pour faire fondre la gélatine. Cette température T1 dépend de la composition exacte de l'encre et doit être déterminée par des essais simples : pour le domaine le domaine de composition le plus préférentiel indiqué ci-dessus une température T1 de l'ordre de 28°C à 29° peut convenir. Cette impression peut être réalisée par tout moyen approprié, par exemple à l'aide d'une seringue monté sur un chariot, sur lequel la seringue se déplace le long d'un axe, et le chariot se déplace dans un sens orthogonal audit axe. Le substrat est posé sur une table ou plateforme. Un déplacement selon l'axe de la hauteur peut être prévu soit pour le chariot porteur de la seringue soit pour la table ou plateforme porteuse du substrat.

Le substrat est avantageusement refroidi afin que la gélatine se solidifie immédiatement ; Cette température T2 du substrat dépend de la composition et température T1 de l'encre et de la vitesse de son dépôt ; elle peut être déterminée par des essais simples. Dans un autre mode de réalisation l'ensemble d'impression se trouve dans une chambre froide, c'est-à-dire à la température T2, optionnellement la seringue peut être chauffée à la température T1. Ce mode de réalisation est avantageux pour permettre la fabrication de structures plus épaisses ou de forme plus complexe, pour lesquelles la conduction thermique à travers la couche d'encre qui vient d'être déposée ne permet pas à la couche d'encre en cours de dépôt un refroidissement suffisamment rapide.

Dans une sixième étape on consolide l'objet imprimé brut en le traitant avec la solution de polymérisation (quatrième solution), de préférence par immersion totale ; ainsi on obtient un objet dit « objet solidifié ». Le temps de contact entre l'objet imprimé brut et la solution de polymérisation est de préférence d'au moins quinze minutes. Lors de cette étape la température T3 de la solution de polymérisation se trouve de préférence au-dessus de la température de fusion de la gélatine. Cela permet à la gélatine de passer en phase aqueuse, et ainsi la plus grande partie de la gélatine est enlevée de l'objet solidifie. On obtient ainsi un objet appelé « précurseur de tissu corporel » qui peut être un « précurseur de substitut cutané ». La température T3 peut être d'environ 37°C.

Les étapes 5 et 6 sont illustrées de manière schématique sur la figure 1.

Dans une sixième étape on incube le précurseur de tissu corporel (qui peut être un précurseur de substitut cutané) pour obtenir un substitut de tissu corporel (par exemple un substitut cutané). Cette incubation se fait en trois phases. Pendant une première phase d'incubation on immerge ledit précurseur de substitut cutané dans un milieu de culture de fibroblastes appropriée, de préférence à une température d'environ 37°C. La durée de cette incubation peut être comprise entre trois et vingt jours (jusqu'à 40 jours avec les kératinocytes) ; une durée comprise entre huit et quinze jours est préférée ; une durée de douze jours est optimale pour les fibroblastes.

A l'issue de cette première phase d'incubation on applique sur la surface du précurseur de substitut cutané une suspension aqueuse de kératinocytes. Une concentration comprise entre 1 et 5 x 10⁵ cellules par cm² convient.

Puis, pendant une deuxième phase d'incubation on immerge le précurseur de substitut cutané dans une solution de culture appropriée, par exemple le milieu de Green ; il faut apporter des nutriments régulièrement (chaque jour de préférence). Cette deuxième phase d'incubation dure entre cinq et dix jours, de préférence environ sept jours. La température est de 37°C.

Et enfin, pendant une troisième phase d'incubation on maintient les précurseurs de substitut cutané à la surface du milieu de culture de différenciation et on les incube pendant une durée comprise entre 15 et 30 jours, de préférence entre 18 et 25 jours, typiquement 21 jours. La température est de 37°C. Ainsi on obtient un substitut cutané. Ledit milieu de différenciation comprend typiquement du DMEM ainsi que des additifs spécifiques ; dans un mode de réalisation avantageux ces additifs sont : hydrocortisone (0,4 µg/mL), insuline (5 µg/mL), albumine bovine (8 mg/mL).

Le procédé de bio-impression selon l'invention permet d'obtenir des objets de tailles centimétriques voir décimétriques. L'imprimante ressemble à une imprimante 3D FDM classique (Fused Deposition Modelling) mais le pousse-seringue remplace alors les extrudeurs plastiques.

La bio-encre utilisée dans le procédé selon l'invention répond à trois objectifs :
- Le maintien de l'hydrogel avec une rhéologie adéquate pendant le processus d'impression (d'extrusion),
- La formation d'un objet polymérisé de manière homogène, qui va conserver sa forme obtenue en 3D,
- Permettre le développement d'un réseau cellulaire en 3D.

Ces trois fonctions ont été validées grâce à l'utilisation des biomatériaux suivants. La gélatine, un polymère à base de collagène ayant une température de transition de phase à 29°C, a été utilisée en tant que composant rhéologique, ce qui donne à la bio-encre sa tenue une fois imprimée sur un substrat refroidi et qui pourra ensuite être éliminé dans les étapes ultérieures du procédé. L'alginate, un polymère à base d'hydrate de carbone avec la capacité de former un hydrogel en présence de calcium, utilisé comme un élément de structure, permet de donner une stabilité mécanique à la bio-encre imprimée une fois la gélatine solubilisée. Le fibrinogène, une glycoprotéine ayant la capacité de former un hydrogel sous l'action de la thrombine, utilisé à la fois comme un élément de construction et de maturation grâce à ses composants d'adhérence cellulaire (motifs RGD).

La figure 6(a) illustre de manière schématique le déroulement du procédé utilisé. On dépose par le procédé selon l'invention un précurseur de substitut cutané comprenant des fibroblastes dermiques normaux humains (NHDF). En immersion totale on incube pendant quatre jours dans un milieu de culture à 37°C pour stimuler la synthèse de matrice extracellulaire. Ensuite on ensemence des kératinocytes normaux humains (NHEK) et laisse incuber encore trois jours pour stimuler la prolifération cellulaire, leur migration et adhésion. Après la septième journée à compter du début de l'incubation on poursuit l'incubation à l'interface air-liquide pour obtenir la différentiation des cellules jusqu'à un total de 21 jours.

L'incubation peut être effectuée dans le puits d'une plaque multi-puits ou en boîte de Pétri. La figure 6(c) montre de manière schématique les conditions d'incubation en immersion (utilisée pour les premiers sept jours) : à gauche dans le puits d'une plaque multi-puits **3,** à droite dans une boîte de culture. Dans la variante « plaque multi-puits », le précurseur de substitut corporel bio-imprimé **1** est placé dans un insert de culture **2** se trouvant dans le puits **3** d'une plaque multi-puits. Le milieu de culture **4** recouvre le précurseur de substitut corporel **1.** Dans la variante « boîte de culture » le précurseur de substitut corporel bio-imprimé **1** est placé dans un bac **8** contenant le milieu de culture **4 ;** un couvercle **5** recouvre le bac **8.**

La figure 6(b) montre de manière schématique les conditions d'incubation en émersion (utilisée à partir du huitième jour): à gauche dans le puits d'une plaque multi-puits **3,** à droite dans une boîte de culture. Dans la variante « plaque multi-puits », le substitut corporel **1** est placé dans un insert de culture **2** dont la surface inférieure touche la surface supérieure du milieu de culture **4** se trouvant dans le puits **3** d'une plaque multi-puits. Dans la variante « boîte de culture » le substitut corporel **1** est placé sur une grille en acier inoxydable **6** qui plonge légèrement dans le milieu de culture **4** contenu dans un bac **8.** La surface supérieure de la grille **6** est recouverte d'une feuille de buvard **7** ; un couvercle **5** recouvre le bac **8.**

L'invention a de nombreuses applications. Le procédé de fabrication d'un substitut de tissu corporel selon l'invention permet de fabriquer des substituts de tissus corporels de différentes natures. En effet, on peut incorporer dans la bio-encre utilisée dans le procédé selon l'invention tous types de cellules vivantes. A titre d'exemple, on peut incorporer dans la bio-encre tous les autres types cellulaires de la peau (notamment : les ADSC (en anglais « Adipose-Derived Stem Cells »), adipocytes et préadipocytes, cellules endothéliales, cellules nerveuses, cellules dendritiques dermiques, cellules de Langerhans, mélanocytes, cellules de Merkel, sébocytes, macrophage; mastocytes, cellules épithéliales des follicules pileux, les fibroblastes de la papille du follicule pileux, les cellules souches pluripotentes induites. Le procédé permet notamment de fabriquer des substituts pour une variété de tissus corporels, et notamment des substituts pour la cornée, la muqueuse orale, l'œsophage, le cartilage, les vaisseaux, la muqueuse vaginale.

D'une manière générale, dans le cadre de la présente invention des cellules vivantes qui sont des cellules primaires isolées de tout tissu ou organe humain ou animal ou des cellules souches animales ou des cellules souches humaines non embryonnaires sont intégrées dans la bio-encre.

Ces cellules peuvent être des cellules souches embryonnaires non-humaines (totipotentes, pluripotentes et tripotentes) ou des cellules différenciées (cellules de la lignée germinale ou cellules somatiques), des cellules primaires isolées de tout tissu ou organe humain ou animal, les cellules non-humaines de la lignée germinale (les gamètes), les cellules somatiques et les cellules souches adultes. Les cellules peuvent provenir notamment de tissu conjonctif ou de soutien (tel que : os, ligament, cartilage, tendon, tissu adipeux), tissu musculaire (tel que : cellules musculaires lisses de la paroi vasculaire, muscle cardiaque, muscle squelettique), tissu nerveux, et les épithélia (tel que : vaisseaux sanguins, canal de Wharton, muqueuses buccales, dos de la langue, voute du palais, oesophage, pancréas, glande surrénale, prostate, foie, thyroïde, estomac, intestin, intestin grêle, rectum, anus, vésicule biliaire, follicule thyroïdien, vaisseau lymphatique, peau, glande sudoripare, mésothélium des cavités corporelles, ovaire, trompe utérine, utérus, endomètre, col de l'utérus (endocol, exocol), vagin, grande lèvres, tubuli recti, rete testis, ductuli efferentes, epididymis, vas deferens, canal éjaculatoire, glande bulbo-urétrale, vésicule séminale, oropharynx, larynx, corde vocale, trachée, bronchioles, cornée, nez, tube contourné proximal du rein, tube distal du rein, bassinet rénal, uretère, vessie urinaire, urètre prostatique).

Il peut s'agir de cellules d'origine mésodermique, ectodermique ou endodermique.

Les cellules humaines ou animales peuvent être sélectionnées dans le groupe formé par : lymphocytes (notamment lymphocyte B, lymphocyte T, lymphocyte T cytotoxique, lymphocyte NKT, lymphocyte T régulateur, lymphocyte auxiliaire), cellules myéloïde, granulocytes, granulocytes basophiles, granulocytes éosinophiles, granulocytes neutrophile, neutrophiles hypersegmentés, monocytes, macrophages, réticulocytes, trombocyte, mastocytes, thrombocytes, megakaryocytes, cellules dendritiques, cellules thyroïdiennes, cellules épithéliales de la thyroïde, cellules parafolliculaires, cellules parathyroïdes, cellules principales de la glande parathyroïde, cellules oxyphiles, cellules surrénales, cellules chromaffines, cellules pinéales, cellules gliales, glioblastes, astrocytes, oligodendrocytes, cellules de la microglie, cellules magnocellulaires neurosécrétrice, cellules stellaires, cellules de Boettcher; cellules pituitaires, gonadotropes, corticotropes, thyrotropes, somatotrope, lactotrophes, cellules pulmonaires (pneumocytes de type I, pneumocytes de type II, cellules de Clara); cellules à goblet, macrophages alveolaires, myocardiocytes, péricytes, cellules gastriques (gastric chief cells, cellules parietales, cellules à goblet, cellules de paneth, cellules G, cellules D, cellules ECL, cellules I, cellules K, cellules S, cellules entéroendocrine, cellules entérochromaffines, cellules APUD), cellules du foie (hépatocytes, cellules de Kupffer), cellules osseuses (ostéoblastes, ostéocytes, ostéoclastes, odontoblastes, cementoblastes, ameloblastes), cellules du cartilage (chondroblastes, chondrocytes), cellules de cheveux (trichocytes), cellules de peau (kératinocytes, adipocytes, fibroblastes, mélanocytes, cellules de nevus), cellules musculaires (myocytes, myoblastes, myotubes), cellules de tendon, cellules rénales (podocytes, cellules juxtaglomerulaires, cellules intraglomérulaires mesangiales, cellules extraglomerulaires mesangiales, cellules de la macula densa), spermatozoides, cellules de Sertoli, cellules de Leydig, ovocytes.

Les cellules peuvent également être isolées à partir d'un tissu malade, par exemple cancéreux.

Par exemple, les cellules peuvent être isolées ou dériver de beaucoup de types de cancers: cancer du sein; cancer du tractus biliaire; cancer de la vessie; cancer du cerveau incluant les glioblastomes et medulloblastomes; cancer cervical; choriocarcinome; cancer du côlon; cancer de l'endomètre; cancer de l'oesophage; cancer gastrique; néoplasie hématologique incluant les leucémies; néoplasie intraépithéliale incluant la maladie de Bowen et la maladie de Paget; cancer du foie; cancer des poumons; lymphomes incluant la maladie d'Hodgkin; neuroblastomes; cancers oraux incluant le carcinome épidermoïde; cancer ovarien incluant ceux provenant des cellules épithéliales, des cellules stromales, des cellules germinales et mésenchymales; cancer du pancréas; cancer de la prostate; cancer rectal; sarcomes incluant leiomyosarcome, rhabdomyosarcome, liposarcome, fibrosarcome, et ostéosarcome; cancer de la peau incluant mélanome, carcinome des cellules de Merkel, sarcome de Kaposi, carcinome basocellulaire, et carcinome épidermoïde; cancer testiculaire incluant les tumeurs germinales comme le seminome, non-seminomae (tératomes, choriocarcinomes), tumeurs stromales, et tumeurs de cellules germinales; cancer de la thyroïde incluant l'adénocarcinome de la thyroïde et carcinome médullaire; le cancer du rein incluant l'adénocarcinome et la tumeur de Wilms.

Les cellules peuvent être des cellules de sang de cordon, des cellules souches, des cellules souches embryonnaires non -humaines, des cellules souches adultes, des cellules souches cancéreuses, des cellules progénitrices, des cellules souches pluripotentes induites, des cellules autologues, des cellules d'isogreffe, des cellules allogéniques, des cellules de xénogreffe et des cellules génétiquement modifiées. Les cellules peuvent être des cellules progénitrices induites. Les cellules peuvent être des cellules isolées d'un sujet, par exemple un sujet donneur, qui a été transfecté avec un gène associé à des cellules souches pour induire une pluripotence dans les cellules. Les gènes associés aux cellules souches peuvent être choisis dans le groupe constitué par Oct3, Oct4, Sox1, Sox2, Sox3, Sox15, Klf1, Klf2, Klf4, Klf5, Nanog, Lin28, C-Myc, L-Myc et N-Myc. Les cellules peuvent être des cellules qui ont été isolées d'un sujet, transfectées avec un gène associé à des cellules souches pour induire la pluripotence et différenciées le long d'une lignée cellulaire prédéterminée.

Ainsi, le substitut de tissu corporel obtenu par le procédé selon l'invention peut être utilisé non seulement comme substitut cutané pour des essais de produits cosmétiques, pharmaceutiques et chimiques, mais également pour des applications cliniques, par exemple en chirurgie réparatrice et reconstructrice. A titre d'exemple, le substitut cutané obtenu par le procédé selon l'invention peut servir comme peau pour les brûlés. Le procédé de fabrication d'un substitut de tissu corporel par dépôt additif selon l'invention permet également de fabriquer des objets de taille complexe, et ainsi on peut fabriquer des substituts d'oreille ou de nez par exemple, en utilisant des cellules prélevées sur le patient auquel est destiné ledit substitut corporel.

D'une manière générale, les substituts corporels obtenus par le procédé selon l'invention peuvent être implantés dans le corps d'un patient (humain ou animal), peuvent servir comme modèle pour étudier des substances d'intérêt (notamment pharmacologiques ou cosmétiques, ou pour caractériser des substances chimiques), ou peuvent servir comme objet modèle pour l'enseignement (notamment pour des travaux pratiques et pour des essais préparatoires en chirurgie). D'une manière avantageuse les substituts corporels obtenus par le procédé selon l'invention peuvent être utilisés pour les applications chirurgicales suivantes : peau et cartilage (oreilles, nez) pour les grand-brûlés, implantation de gencives, œsophage artificiel, uretère et urètre artificiels, cornée.

Par ailleurs, le substitut de tissu corporel obtenu par le procédé selon l'invention peut être utilisé pour caractériser la toxicité, l'efficacité ou la pénétration dans le tissu corporel de produits chimiques divers. Il peut aussi être utilisé pour des essais d'allergènes.

Il est bien entendu que la présente invention peut être appliquée indifféremment aux cellules humaines et aux cellules d'autres d'animaux, notamment d'autres mammifères.

### Exemples

### Les exemples qui suivent illustrent certains aspects de l'invention, mais ne limitent pas sa portée.

### Exemple 1 : Culture et récolte de cellules

Cet exemple illustre un procédé d'amplification et de récolte de cellules (fibroblastes et kératinocytes) qui peuvent ensuite être utilisées dans la fabrication du substitut cutané selon l'invention.

On a isolé des kératinocytes et fibroblastes dermiques à partir d'un *preputium* humain.

Les kératinocytes ont été cultivés sur des fibroblastes humains irradiés selon une technique bien connue de l'homme du métier, en utilisant le milieu de culture connue sous le nom « Green's medium » contenant du DMEM et Ham's F12 (dans un rapport de 3 :1), auquel on a ajouté de l'adénine (24,3 µg/mL), le facteur de croissance épidermique humain (10 ng/mL), de l'hydrocortisone (0,4 µg/mL), de l'insuline (Humulin ^{®}, 5 µg/mL), 2 x 10⁻⁹ M tri-iodo-L-thyronine (5 µg/mL), 10⁻¹⁰ M isoproterenol, de la pénicilline (100 U/mL), de la streptomycine (100 µg/mL) et 10% de sérum de veau fœtal. On a utilisé les kératinocytes récoltés lors des passages 2, 3 et 4.

Les fibroblastes ont été cultivés dans un milieu approprié comportant du DMEM, 20% de sérum de veau nouveau-né et des antibiotiques, à 37°C dans une atmosphère comportant 5% CO₂. On a utilisé les fibroblastes récoltés lors des passages 5, 6, 7 et 8.

### Exemple 2 : Dépôt par technique additive (compris dans le procédé selon l'invention)

On a préparé une première solution aqueuse de gélatine en dissolvant une poudre de gélatine à 20% m/v dans une solution de NaCl à 0,9% m/v. On a préparé une deuxième solution aqueuse d'alginate en dissolvant de la poudre d'alginate (Very Low Viscosity) à 4% m/v dans une solution de NaCl à 0,9% m/v. On a préparé une troisième solution aqueuse de fibrinogène à 8% m/v dans laquelle on a introduit des fibroblastes en suspension (obtenus selon l'exemple 1) en une concentration cellulaire de 2 millions cellules/mL.

Ensuite on a mélangé ces trois solutions de manière à obtenir un mélange (dit « bio-encre ») qui contient 50 vol-% de la première solution (gélatine), 25 vol-% de la deuxième solution (alginate) et 25 vol-% de la troisième solution (fibroblastes repris dans le fibrinogène).

On a préparé une solution aqueuse de polymérisation comprenant calcium 3% m/v et de thrombine à une concentration finale de 20 U/mL.

Ladite bio-encre présente une transition visqueuse à 29°C. Elle a été chauffée à une température d'environ 30°C et utilisée à cette température pour le dépôt additif selon une technique de dépôt de type connue, à travers une seringue équipé d'un pousse-seringue pour pouvoir contrôler le débit. Le substrat se trouvait à une température d'environ 4°C, et de ce fait l'encre déposée s'est solidifiée immédiatement. A partir d'un boudin d'un diamètre de 300 µm on a déposé plusieurs couches d'une épaisseur totale d'environ 10 mm sur une surface de l'ordre de quelques centimètres carrés. On a ainsi obtenu des objets plats qui comprenaient une répartition homogène de fibroblastes, à raison de 2,5 x 10⁵ fibroblastes par cm².

Ensuite chaque objet imprimé brut ainsi déposé a été trempé dans la solution de polymérisation pour réticuler l'alginate et coaguler le fibrinogène maintenue à une température de 37°C pour polymériser l'alginate et coaguler le fibrinogène. L'objet plat ainsi obtenu est appelé ici « précurseur de substitut cutané ».

### Exemple 3 : Maturation du précurseur de substitut cutané (comprise dans le procédé selon l'invention)

On a incubé les précurseurs de substitut cutané pendant 12 jours dans un milieu de culture de fibroblastes comprenant 1 mM d'acide ascorbique 2-phosphate ; ils ont été alimentés chaque jour. Après douze jours on a appliqué sur la surface des précurseurs de substitut cutané des kératinocytes en une concentration de 2,5 x 10⁵ cellules par cm².

Les précurseurs de substitut cutané ont été incubés pendant une première période d'incubation de sept jours en immersion dans le milieu de Green comme décrit ci-dessus, avec une concentration de 1mM d'acide ascorbique 2-phosphate et des antibiotiques ; ils ont été alimentés chaque jour.

Ensuite les précurseurs de substitut cutané ont été incubés pendant une deuxième période d'incubation de 21 jours, maintenus à la surface du liquide, dans un milieu de différenciation contenant du DMEM avec de l'hydrocortisone (0,4 µg/mL), de l'insuline (5 µg/mL), de l'acide ascorbique 2-phosphate et des antibiotiques ; le milieu de différentiation comportait de l'albumine de sérum bovin à raison de 8 mg/mL.

On a ainsi obtenu des substituts cutanés. Ils peuvent être utilisés pour effectuer des essais de produits cosmétiques ou chimiques.

La figure 2 montre une coupe transversale à travers un tel substitut cutané. On aperçoit l'épiderme comportant à sa périphérie une véritable couche cornée, et comportant des kératinocytes. La jonction dermo-épidermique est nette. Le derme comprend des fibroblastes (sur la figure 2 on voit uniquement les noyaux, entourés par une ligne noire). Dans le derme on aperçoit des pores ; ceux-ci se replissent progressivement de matrice extracellulaire sécrétée par les fibroblastes.

La figure 3 montre les filaments intermédiaires du cytosquelette des fibroblastes dans le substitut de derme (marquage immuno-histochimique de la vimentine). On note la forme tridimensionnelle du cytosquelette, qui ressemble à celle dans un derme naturel sain.

### Exemple 4 : Divers essais qui ne donnent pas satisfaction

Cet exemple rassemble des tentatives de fabrication de substituts de derme selon des procédés selon l'état de la technique ou selon des procédés nouveaux, qui ne donnent pas satisfaction.

### Exemple 4a : Encre photopolymérisable à base de PEG diacrylate

On a préparé un précurseur de substitut cutané avec une bio-encre à base de PEG-DA (polyéthylène glycol diacrylate) photopolymérisable en utilisant un procédé similaire à celui décrit en relation avec la présente invention. Le photoinitiateur était le Irgacure ^{™} 819 (Bis(2,4,6-triméthylbenzoyl)-phénylphosphine oxide, société CIBA). Après incubation on a constaté un taux de mortalité cellulaire de 100% après 2 jours. Sans vouloir être liés par cette théorie, les inventeurs pensent que ce n'est pas le photoinitiateur en tant que tel qui est cytotoxique mais les radicaux libres dont il promeut la génération.

### Exemple 4b : Encre utilisée dans le procédé selon l'invention mais sans fibrinogène

On a réalisé une bio-encre sans fibrinogène, mais qui était conforme à l'invention pour tous les autres ingrédients, avec des fibroblastes murins fluorescents de la lignée NIH3T3. On a préparé à partir de cette bio-encre un précurseur de substitut cutané en utilisant les étapes du procédé selon l'invention. Dans ce cas, malgré une bonne tenue du gel, les cellules ne se développaient pas de manière satisfaisante au cours de l'incubation du fait de l'absence de motif d'adhésion cellulaire au sein du gel. Comme cela ressort de la figure 4, les cellules ne s'étalent pas et finissent par mourir. Ce substitut cutané n'est donc pas utilisable pour des essais de produits cosmétiques, pharmaceutiques et chimiques.

### Exemple 4c : Encre trop fluide

On a utilisé le procédé selon l'invention, en utilisant différents types d'alginates qui se distinguaient notamment par leur viscosité : « Low Viscosity » et « High Viscosity » au lieu de « Very Low Viscosity ». On a constaté qu'avec l'encre « High Viscosity » on n'obtient pas une extrusion satisfaisante. Avec l'encre « Low Viscosity » on peut régler le procédé pour obtenir des objets imprimés bruts, mais ce mode de réalisation est propice à l'instabilité de l'extrusion, et n'est pas préféré.

### Exemple 4d : Autres causes d'échecs

Des échecs (formation d'un précurseur de substitut cutané de mauvaise morphologie et/ou qualité) ont été observés dans les cas suivants :
- Temps de contact trop court entre la bio-encre après son application et la solution de polymérisation (inférieur à environ quinze minutes) ;
- Défaut d'immersion totale du dépôt de bio-encre pendant sa polymérisation ;
- Vieillissement de la solution de polymérisation.

### Exemple 5 : Fabrication d'une peau totale avec les 3 couches de la peau : hypoderme, derme et épiderme (procédé selon l'invention).

Avec le même procédé il est possible d'imprimer les trois couches de la peau avec trois seringues différentes comportant la bio-encre et des cellules différentes :
Une première seringue contient des pré-adipocytes et/ou des adipocytes matures dilués dans la bio-encre. Cet hypoderme est imprimé en plusieurs couches. Il est imprimé en premier pour former la couche la plus profonde de la peau.

La deuxième seringue contient les fibroblastes dans la bio-encre comme décrit dans le procédé. Cette partie de derme est imprimée en plusieurs couches à la surface de l'hypoderme préalablement imprimé

La troisième seringue contient des kératinocytes dilués dans une bio-encre un peu différente. Cet épiderme est imprimé en une seule ou plusieurs couches à la surface de la couche du derme. On peut ajouter à tout moment à la suspension de kératinocytes, des mélanocytes en proportion 1/10 à 1/2, pour obtenir des peaux pigmentées.

### Exemple 6 : Bio-encres, avec charge en cellules variable

On a préparé trois bio-encres qui ne se distinguaient que par leur charge en cellules vivantes (fibroblastes humains) : 50 000 cellules par mL de bio-encre, 100 000 cellules par mL de bio-encre et 200 000 cellules par mL de bio-encre. Avec ces bio-encres on a réalisé des précurseurs de substituts dermiques par le procédé selon l'invention. On a laissé incuber les précurseurs de substituts dermiques imprimés pendant 4 jours à 37°C sous 5% volumiques de CO₂, et on a déterminé la fraction de cellules vivantes par un test de bleu Alamar^{™} (mesure de l'absorbance à 570 nm et 600 nm, la quantité de cellules vivantes résulte du rapport entre ces deux valeurs d'absorbance). On constate que la bio-encre permet la survie des fibroblastes dans le substitut dermique, et que la concentration de ces cellules vivantes dans le précurseur de substitut dermique est proportionnelle à la concentration des cellules vivantes dans la bio-encre (voir la figure 5a).

Pour une densité cellulaire donnée (en l'occurrence 100 000) on a déterminé la quantité de cellules vivantes après 7, 10 et 14 jours de culture dans les conditions d'incubation indiquées ci-dessus. Les résultats sont montrés sur la figure 5(b). On voit que ces cellules prolifèrent dans le substitut cutané avec le temps.

Après 14 jours, on a procédé à une coloration du substitut cutané par le DAPI (qui colore les noyaux des cellules vivantes) et le iodure de propidium (qui colore les noyaux des cellules mortes). La figure 5(c) montre la coloration au DAPI (voile gris et les quelques zones colorés au iodure de propidium (taches blanches) sur une coupe de microscopie optique. La figure 5(d) montre une expérience de contrôle d'un échantillon identique dont les cellules ont été tuées par un traitement au sodium dodécyl sulfate (0,5%) : on voit la coloration au iodure de propidium en blanc.

### Exemple 7 : Préparation d'un substitut cutané selon l'invention

On a préparé un substitut cutané, et plus particulièrement selon le procédé illustré sur la figure 6. La figure 7(a) montre une coupe transversale à travers un substitut de derme obtenu par le procédé selon l'invention ; cette figure a été obtenue par coloration HPS (hématoxyline - phloxine - safran). La figure 7(b) montre une coupe transversale à travers le même substitut de derme (épaisseur 5 µ) ; la coloration a été obtenue au trichrome de Masson. On distingue dans le derme bio-imprimé les noyaux des fibroblastes (N) est la matrice extracellulaire (MEC) néosynthétisée par les fibroblastes. Les figures 7(c), 7(d) et 7(e) montrent, respectivement, un marquage immuno-histologique spécifique pour le collagène I (figure 7(c)), le collagène V (figure 7(d)) et la fibrilline (figure 7(e)) dans le derme bio-imprimé selon le procédé de l'invention (dans une zone à cheval sur l'épiderme et le derme), alors que les figures 7(f), 7(g) et 7(h) montrent à titre de comparaison des images correspondantes, obtenues dans les mêmes conditions, d'une peau humaine (dans ces six figures certaines lignes ont été redessinées en pointillé pour les rendre plus visibles).

On a également détecté la vimentine et l'élastine (non montrés sur les figures).

Les figures 7(i) et 7(j) montent, respectivement, un marquage immuno-histologique spécifique pour la laminine 332 (figure 7(i)) et le collagène VII (figure 7(j)) dans le derme bio-imprimé selon le procédé de l'invention (dans une autre zone à cheval sur l'épiderme et le derme), alors que les figures 7(k) et 7(l) montrent à titre de comparaison des images correspondantes, obtenues dans les mêmes conditions, d'une peau humaine.

Les figures 8(a) et 8(b) montrent des agrandissements de zones du derme bioimprimé de la figure 7(c), à savoir un fibroblaste (figure 8(a)) - les symboles N, Re et Rb désignent, respectivement, le noyau, le réticulum et le ribosome - et du collagène néosynthétisée (figure 8(b)). Dans la figure 8(a) la barre noire en bas à gauche correspond à une longueur de 0,5 µm, dans la figure 8(b) à 200 nm.

Les figures 8(c) et 8(d) montrent des détails de la figure 8(b), à savoir le collagène strié (figure 8(c)) et un dépôt d'élastine soluble (figure 8(d)).

La figure 9 montre une image du derme bio-imprimé selon l'invention. Les repères numériques désignent des caractéristiques ultrastructurales, à savoir : les filaments intermédiaires de cyrokératine **10,** l'hémidesmosome **11,** la *lamina lucida* **12,** la *lamina densa* **13,** les fibrilles d'ancrage **14,** les fibres de collagène **15.** La longueur de la barre verticale en bas à droite correspond à 0,2 µm.

Les figures 10(a), 10(b) et 10(c) montent, respectivement, un marquage immunohistologique spécifique pour la cytokératine 10 (figure 10(a)), la filaggrine (figure 10(b)) et la loricrine (figure 10(c)) dans le derme bio-imprimé selon l'invention (dans une autre zone à cheval sur l'épiderme et le derme), alors que les figures 10(d), 10(e) et 10(f) montrent à titre de comparaison des images correspondantes, obtenues dans les mêmes conditions, d'une peau humaine.

La figure 11(a) montre une autre image ultrastructurale du substitut de derme ; cette zone montre la couche cornée *(stratum corneum).* Les figures 11(b), (c), (d) et (e) montrent des détails, à savoir : les cornéodesmosomes (figure 11(b), le desmosome (figure 11(c)), les granules de kératohyaline (figure 11(d)), les corps lamellaires d'Odland (figure 11(e)).

Cet exemple montre que le substitut de derme obtenu par le procédé selon l'invention est non seulement morphologiquement similaire à la peau humaine saine, mais exprime aussi tous les biomarqueurs reliés à la différentiation et prolifération épidermique (tels que : cytokératine 10, filaggrine), est capable d'agir comme une barrière fonctionnelle (voir notamment l'expression de loricrine), et exprime des protéines que l'on trouve habituellement dans la matrice extracellulaire (notamment : collagène I, collagène V, fibrilline, vimentine, élastine) : cela illustre la grande proximité morphologique, histologique et fonctionnelle du substitut de derme obtenu selon l'invention avec le derme humain.

### Exemple 8 : Substitut corporel avec cellules endothéliales humaines

On a préparé deux bio-encres, chargées, respectivement, de 200 000 et 400 000 cellules endothéliales dermiques humaines par millilitre. On a déposé un précurseur de substitut corporel, et on a déterminé la viabilité des cellules après une incubation de 4, 8 et 14 jours par le test au bleu Alamar^{™}, décrit ci-dessus. La figure 12 montre la prolifération des cellules endothéliales imprimées en fonction des densités cellulaires initiales.

### Exemple 9 : Substitut corporel avec fibroblastes de cornée humaines

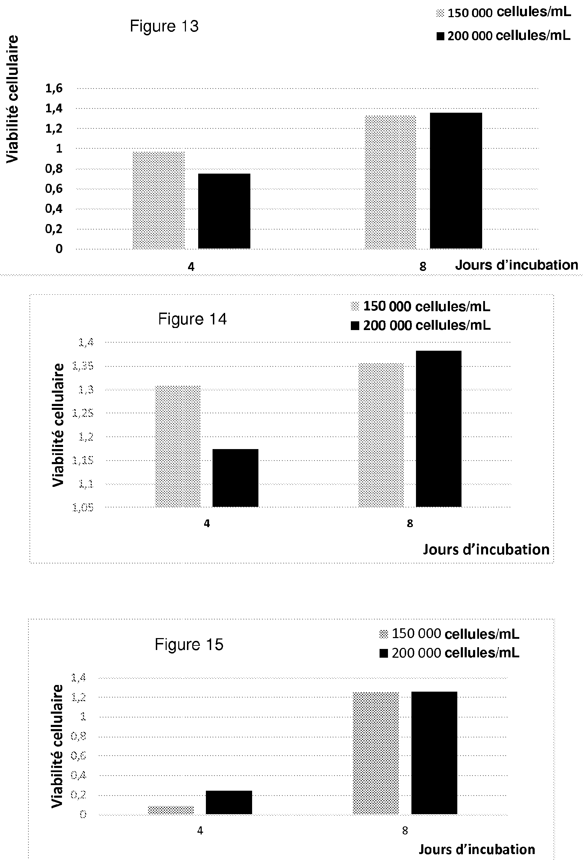

On a préparé deux bio-encres, chargées, respectivement, de 150 000 et 200 000 fibroblastes de cornée humains par millilitre. On a déposé un précurseur de substitut corporel, et on a déterminé la viabilité des cellules après une incubation de 4 et 8 jours par le test au bleu Alamar^{™}, décrit ci-dessus. La figure 13 montre la prolifération des cellules imprimées en fonction des densités cellulaires initiales.

### Exemple 10 : Substitut corporel avec fibroblastes de muqueuse orale humaines

On a préparé deux bio-encres, chargées, respectivement, de 150 000 et 200 000 fibroblastes de muqueuse orale humains. On a déposé un précurseur de substitut corporel, et on a déterminé la viabilité des cellules après une incubation de 4 et 8 jours par le test au bleu Alamar^{™}, décrit ci-dessus. La figure 14 montre la prolifération des cellules imprimées en fonction des densités cellulaires initiales.

### Exemple 11 : Substitut corporel avec fibroblastes de papille dermique du follicule pileux humaine

On a préparé deux bio-encres, chargées, respectivement, de 150 000 et 200 000 fibroblastes de papille dermique du follicule pileux humaine. On a déposé un précurseur de substitut corporel, et on a déterminé la viabilité des cellules après une incubation de 4 et 8 jours par le test au bleu Alamar^{™}, décrit ci-dessus. La figure 15 montre la prolifération des cellules imprimées en fonction des densités cellulaires initiales.

### Exemple 12 : Bio-encres avec diverses cellules vivantes

On a préparé quatre bio-encres, chargées, respectivement, de 100 000, 200 000, 400 000 et 600 000 cellules souches adipeuses par millilitre de bio-encre, d'une manière similaire de celle décrite à l'exemple 6, et on a préparé un précurseur de substitut corporel. On constate que la bio-encre permet la survie des cellules souches adipeuses dans le précurseur de substitut corporel, et que la concentration de ces cellules vivantes dans ledit précurseur proportionnelle à la concentration des cellules vivantes dans la bio-encre (voir la figure 16a).

De manière analogue, on a préparé quatre bio-encres, chargées, respectivement, de 100 000, 200 000, 400 000 et 600 000 de pré-adipocytes par millilitre de bio-encre, et on a préparé un précurseur de substitut corporel. On constate que la bio-encre permet la survie des préadipocytes souches adipeuses dans le précurseur de substitut corporel, et que la concentration de ces cellules vivantes dans ledit précurseur proportionnelle à la concentration des cellules vivantes dans la bio-encre (voir la figure 16b). Les figures 16(c) et 16(d) ont été expliquées précédemment (section « Figures »).

### Exemple 13 : Fabrication d'un précurseur de substitut d'une oreille

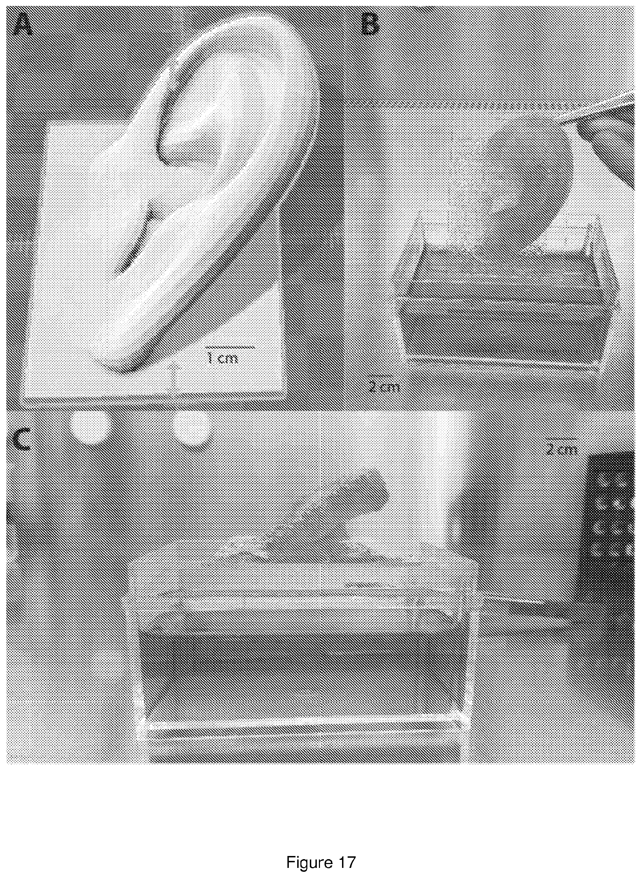

En utilisant le procédé selon l'invention, on a préparé un précurseur de substitut d'une oreille humaine, dont la plus grande dimension était d'environ 8 cm. La bio-encre a été préparée avec 10% (w/v) de gélatine bovine (n° CAS 9000-70-8) fournie par Sigma Aldrich (France), 0,5% (w/v) d'alginate (very low viscosity, n° CAS 9005-38-3) fourni par Aesar (France), et 2% (w/v) de fibrinogène (n° CAS 9001-32-5) fourni par Sigma Aldrich (France) à une température de 37°C.Juste avant l'impression 3D, on a ajouté des cellules fraîchement trypsinisées à raison de 1 x 10⁶ cellules par millilitre de bio-encre. La bio-encre homogénéisée a été transférée dans une seringue ; cette seringue remplie a été stockée pendant 15°C à 37°C afin d'obtenir les propriétés rhéologiques souhaitées. Le diamètre de la micropipette était 200 µm. Le substrat se trouvait à température ambiante. L'objet imprimé brut (figure 17, image 1) a été traité pendant 30 minutes dans une solution (100 mM) de CaCl₂ en présence de thrombine (20 U/mL, n° CAS 9002-04-4), voir la figure 17 (images B et C).

### Exemple 14 : Rhéologie du procédé de dépôt additif

Pour une bio-encre on a caractérisé la viscosité à l'aide d'un viscosimètre rotationnel (AR 2000, société TA Instruments) avec une géométrie cône / plan (25 mm) en utilisant le mode balayage du taux de cisaillement (entre 0,1 et 100 s⁻¹) à 28°C, cette température représentant la température lors du dépôt additif. On a calculé la contrainte de cisaillement selon l'équation *τ_{w} = η γ̇_{w}* où *τ_{w}* [Pa], *γ̇_{w}* [s⁻¹] and *η* [Pa.s] représentent, respectivement, la contrainte de cisaillement, le taux de cisaillement sur la paroi w de la buse d'extrusion et la viscosité de la bio-encre.

On calcule le taux de cisaillement à partir de l'équation de Poiseuille pour un débit de bio-encre fixe de Q = 0,183 mm³.s-¹ selon l'équation : ${\dot{γ}}_{w}^{t , b} = \frac{3 n + 1}{n} \frac{Q}{{πR}_{t , b}^{3}}$

A partir du rayon (R), du taux de cisaillement (*γ̇_{w}*) et de la viscosité (*η*), on a calculé la contrainte de cisaillement (*τ_{w}*) après l'entrée (t) et à la sortie (b) de la buse ont été déterminés.

**Tableau 1:**

| Caractérisation rhéologique du procédé d'extrusion d'une bio-encre à travers une buse frustro-conique (longueur 15 mm) avec un débit de Q = 0,183 mm³.s-¹. | | |
|---|---|---|
| Paramètres | Entrée (*t*) | Sortie (*b*) |
| Rayon [mm] | *Rₜ* = 1,50 ${\dot{γ}}_{w}^{t} = 0 , 12$ | *R_{b}* = 0,20 ${\dot{γ}}_{w}^{b} = 51 , 78$ |
| Taux de cisaillement [s⁻¹] | | |
| Viscosité [Pa.s] | *η^{t}* = 94,54 ${τ}_{w}^{t} = 11 , 61$ | *η^{b}* = 0,97 ${τ}_{w}^{b} = 50 , 48$ |
| Contrainte de cisaillement [Pa] | | |

On note qu'avec cette bio-encre et dans des conditions opératoires réalistes, on trouve une contrainte de cisaillement de l'ordre de 50 Pa. Cela explique l'excellente viabilité cellulaire constatée dans les exemples précédents, sachant qu'il a été rapporté dans l'état de la technique que la viabilité cellulaire diminue pour de nombreux types de cellules lorsqu'elles sont soumises à un cisaillement avec une contrainte de cisaillement d'environ 5 000 Pa. Cette bio-encre peut donc être utilisée pour des procédés de fabrication d'objets bidimensionnels ou tridimensionnels par des techniques de dépôt additif, notamment par extrusion, en utilisant des seringues et orifices disponibles dans le commerce.

### Exemple 15 : Substitut de tissu corporel réalisé avec une bio-encre chargée de cellules pathologiques, pour essais pharmacologiques

En utilisant le procédé selon l'invention, on a réalisé un substitut de tissu cutané chargé de cellules pathologiques, à savoir des fibroblastes et des kératinocytes prélevés sur des patients atteints de dermatite atopique. Ce substitut de tissu cutané a été utilisé comme modèle d'investigation pour étudier les mécanismes moléculaires de cette maladie, et pour étudier l'effet de diverses préparations pharmaceutiques et cosmétiques, notamment sous forme topique (crème contenant des principes actifs) ou systémique (le principe actif se trouvant en solution dans le milieu de culture).

## Revendications

1. Procédé de fabrication d'un substitut de tissu corporel, dans lequel :
(i) on approvisionne une bio-encre ;
(ii) on approvisionne une solution aqueuse, dite « solution de polymérisation », comprenant entre 1% à 5% massiques d'ions de calcium et entre 2 U/mL et 40 U/mL, et de préférence entre 5 U/mL et 40 UmL, et encore plus préférentiellement entre 10 U/mL et 30 U/mL, de thrombine ;
(iii) on porte ladite bio-encre à une température **T1** supérieure à son point de gélification et on la dépose sur un substrat se trouvant à une température T2 inférieure au point de gélification de ladite bio-encre, où elle gélifie pour former un objet de forme tridimensionnelle contrôlée dit « objet imprimé brut », sachant que de manière préférée T1 est compris entre 27°C et 32°C et T2 est compris entre 4°C et 20°C ;
(iv) on traite ledit objet imprimé brut avec ladite solution de polymérisation pour consolider ledit objet imprimé brut en un substitut de tissu corporel ;
(v) on incube ledit précurseur de substitut corporel dans un milieu de culture cellulaire ;
dans lequel la bio-encore approvisionnée à l'étape (i) est obtenue par un procédé de fabrication dans lequel :
(a) On approvisionne une première solution aqueuse comprenant entre 5% et 40% massiques de gélatine, de préférence entre 6% et 30% massiques, et entre 0 % et 5% massiques de NaCl ;
(b) On approvisionne une seconde solution aqueuse comprenant entre 1% et 12% massiques d'alginate, de préférence entre 1% et 8% massiques, et entre 0% et 5% de NaCl ;
(c) On approvisionne une troisième solution aqueuse comprenant entre 1% et 15% massiques de fibrinogène, de préférence entre 3% et 15% massiques, et des cellules vivantes en suspension ;
(d) On créé un mélange comprenant :
environ 35% à 65% volumiques de la première solution ;
environ 15% à 35% volumiques de la seconde solution ;
environ 15% à 35% volumiques de la troisième solution ;
ces proportions étant choisies de manière à s'additionner à 100%,
et dans lequel:
- l'ordre des étapes (a),(b) et (c) est indifférent,
- la teneur en NaCl est choisie de manière à ce que dans ladite première solution et ladite seconde solution réunies elle soit comprise entre 0,2% et 5% massiques, de préférence entre 0,2 et 3% massiques, et encore plus préférentiellement entre 0,4% et 2% massiques,
- lesdites cellules vivantes sont des cellules primaires isolées de tout tissu ou organe humain ou animal ou des cellules souches animales ou des cellules souches humaines non embryonnaires.

2. Procédé de fabrication d'un substitut de tissu corporel selon la revendication 1, **caractérisé en ce que** lesdites cellules vivantes de la bio-encore approvisionnée à l'étape (i) sont des cellules primaires isolées à partir d'un tissu conjonctif ou de soutien, d'un tissu musculaire, d'un tissu nerveux ou d'épithélia.

3. Procédé de fabrication d'un substitut de tissu corporel selon la revendication 1, **caractérisé en ce que** lesdites cellules vivantes de la bio-encore approvisionnée à l'étape (i) sont sélectionnées dans le groupe formé par les cellules de sang de cordon, les cellules souches embryonnaires non humaines, les cellules souches adultes, les cellules souches cancéreuses, les cellules progénitrices, les cellules souches pluripotentes induites.

4. Procédé de fabrication d'un substitut de tissu corporel selon l'une quelconque des revendications 1 à 3, dans lequel le traitement dudit objet imprimé brute avec ladite solution de polymérisation est effectué par trempage, à une température T3 supérieure à T1 et de préférence comprise entre 35°C et 38°C.

5. Procédé de fabrication d'un substitut de tissu corporel selon l'une quelconque des revendications 1 à 4, dans lequel
∘ ledit précurseur de tissu corporel est un précurseur de substitut cutané, et
∘ ladite bio-encre comprend des cellules vivantes en suspension qui sont des fibroblastes ;
∘ ladite incubation est effectuée à une température comprise entre 36°C et 38°C et comprend une première phase d'incubation comprise entre huit et quarante jours, et une deuxième phase d'incubation entre cinq et dix jours, sachant qu'entre la première et la deuxième phase on dépose sur la surface dudit substitut cutané une suspension aqueuse de kératinocytes.

6. Procédé de fabrication d'un substitut de tissu corporel selon la revendication 5, dans lequel ledit objet imprimé brut comprend une surface supérieure sensiblement plate et présente une répartition homogène de fibroblastes, qui est comprise entre 0,2 et 10 x 10⁵, de préférence entre 0,2 et 2 x 10⁵, fibroblastes par cm² de surface supérieure plate.

7. Procédé de fabrication d'un substitut de tissu corporel selon la revendication 5 ou 6, dans lequel la concentration de fibroblastes est comprise entre 0,6 et 12 x 10⁵ fibroblastes par cm³ de bio-encre, et de préférence entre 1 et 7 x 10⁵ fibroblastes par cm³ de bio-encre, et encore plus préférentiellement entre 1 et 5 x 10⁵ fibroblastes par cm³ de bio-encre.

8. Procédé de fabrication d'un substitut de tissu corporel selon l'une quelconque des revendications 5 à 7, dans lequel la quantité de kératinocytes déposées est comprise entre 0,05 et 50 x 10⁵, de préférence entre 0,2 et 20 x 10⁵, et encore plus préférentiellement entre 0,5 et 10 x 10⁵, kératinocytes par cm² de surface supérieure plate.

9. Procédé de fabrication d'un substitut de tissu corporel selon l'une quelconque des revendications 5 à 8, dans lequel ledit substitut de tissu corporel est un substitut cutané, et dans lequel procédé :
(i) Dans une première étape, on imprime un hypoderme en plusieurs couches avec une bio-encre telle que définie dans la revendication 1 comprenant des pré-adipocytes et/ou des adipocytes matures,
(ii) Dans une deuxième étape on imprime en plusieurs couches un derme avec une bio-encre telle que définie dans la revendication 1 comprenant des fibroblastes,
(iii) Dans une troisième étape on imprime un épiderme en une ou plusieurs couches avec une bio-encre telle que définie dans la revendication 1 comprenant des kératinocytes.

## Patentansprüche

1. Verfahren zur Herstellung eines Ersatzes für Körpergewebe, bei dem:
(i) eine Biotinte bereitgestellt wird;
(ii) eine als "Polymerisationslösung" bezeichnete, wässrige Lösung bereitgestellt wird, die zwischen 1 und 5 Gew.-% Kalziumionen und zwischen 2 U/ml und 40 U/ml, vorzugsweise zwischen 5 U/ml und 40 U/ml und noch bevorzugter zwischen 10 U/ml und 30 U/ml Thrombin umfasst;
(iii) die Biotinte auf eine Temperatur T1 oberhalb ihres Gelierungspunkts gebracht und auf ein Substrat aufgebracht wird, das sich bei einer Temperatur T2 unterhalb des Gelierungspunkts der Biotinte befindet, bei der sie geliert, um ein Objekt mit kontrollierter dreidimensionaler Form zu bilden, das als "rohes Druckobjekt" bezeichnet wird, wobei T1 vorzugsweise zwischen 27 °C und 32 °C und T2 zwischen 4 °C und 20 °C liegt;
(iv) das rohe Druckobjekt mit der genannten Polymerisationslösung behandelt wird, um dieses rohe Druckobjekt zu einem Ersatz für Körpergewebe zu konsolidieren;
(v) dieser Vorläufer des Ersatzes für Körpergewebe in einem Zellkulturmedium inkubiert wird;
wobei die in Schritt (i) bereitgestellte Biotinte durch ein Herstellungsverfahren erzielt wird, bei dem:
(a) Eine erste wässrige Lösung bereitgestellt wird, die zwischen 5 und 40 Gew.-%, vorzugsweise zwischen 6 und 30 Gew.-% Gelatine, und zwischen 0 und 5 Gew.-% NaCl umfasst;
(b) Eine zweite wässrige Lösung bereitgestellt wird, die zwischen 1 und 12 Gew.-%, vorzugsweise zwischen 1 und 8 Gew.-% Alginat, und zwischen 0 und 5 Gew.-% NaCl umfasst;
(c) Eine dritte wässrige Lösung bereitgestellt wird, die zwischen 1 und 15 Gew.-%, vorzugsweise zwischen 3 und 15 Gew.-% Fibrinogen, und lebende Zellen in Suspension umfasst;
(d) Ein Gemisch hergestellt wird, das Folgendes umfasst:
etwa 35 bis 65 Vol.-% der ersten Lösung;
etwa 15 bis 35 Vol.-% der zweiten Lösung; etwa 15 bis 35 Vol.-% der dritten Lösung;
wobei diese Anteile so gewählt werden, dass sie sich zu 100 % addieren,
und bei dem:
- die Reihenfolge der Schritte (a), (b) und (c) gleichgültig ist,
- der NaCl-Gehalt so gewählt wird, dass er in der ersten Lösung und der zweiten Lösung zusammen zwischen 0,2 und 5 Gew.-%, vorzugsweise zwischen 0,2 und 3 Gew.-% und noch bevorzugter zwischen 0,4 und 2 Gew.-% liegt,
- die lebenden Zellen Primärzellen sind, die von sämtlichem menschlichen oder tierischen Gewebe oder Organ bzw. sämtlichen tierischen Stammzellen oder nicht-embryonalen menschlichen Stammzellen isoliert sind.

2. Verfahren zur Herstellung eines Ersatzes von Körpergewebe gemäß Anspruch 1, **dadurch gekennzeichnet, dass** die lebenden Zellen der in Schritt (i) bereitgestellten Biotinte Primärzellen sind, die aus Binde- oder Stützgewebe, Muskelgewebe, Nervengewebe oder Epithelien isoliert wurden.

3. Verfahren zur Herstellung eines Ersatzes für Körpergewebe gemäß Anspruch 1, **dadurch gekennzeichnet, dass** die lebenden Zellen der in Schritt (i) bereitgestellten Biotinte aus der Gruppe ausgewählt werden, die aus Nabelschnurblut, nicht-menschlichen embryonalen Stammzellen, adulten Stammzellen, Krebsstammzellen, Vorläuferzellen und induzierten pluripotenten Stammzellen gebildet wird.

4. Verfahren zur Herstellung eines Ersatzes für Körpergewebe gemäß einem der Ansprüche 1 bis 3, wobei die Behandlung des rohen Druckobjekts mit der Polymerisationslösung durch Eintauchen bei einer Temperatur T3, die höher als T1 ist und vorzugsweise zwischen 35 °C und 38 °C liegt, erfolgt.

5. Verfahren zur Herstellung eines Ersatzes für Körpergewebe gemäß einem der Ansprüche 1 bis 4, wobei
- der Vorläufer von Körpergewebe ein Vorläufer von Hautersatz ist, und
- die Biotinte lebende Zellen in Suspension umfasst, die Fibroblasten sind;
- die Inkubation bei einer Temperatur zwischen 36 °C und 38 °C erfolgt und eine erste Inkubationsphase zwischen acht und vierzig Tagen und eine zweite Inkubationsphase zwischen fünf und zehn Tagen umfasst, wobei zwischen der ersten und der zweiten Phase eine wässrige Suspension von Keratinozyten auf der Oberfläche des Hautersatzes abgelagert wird.

6. Verfahren zur Herstellung eines Ersatzes für Körpergewebe gemäß Anspruch 5, wobei das genannte rohe Druckobjekt eine im Wesentlichen flache Oberfläche umfasst und eine homogene Verteilung von Fibroblasten aufweist, die zwischen 0,2 und 10 x 10⁵, vorzugsweise zwischen 0,2 und 2 x 10⁵, Fibroblasten pro cm2 der oberen flachen Oberfläche liegt.

7. Verfahren zur Herstellung eines Ersatzes für Körpergewebe gemäß Anspruch 5 oder 6, wobei die Konzentration von Fibroblasten zwischen 0,6 und 12 x 10⁵ Fibroblasten pro cm³ Biotinte, vorzugsweise zwischen 1 und 7 x 10⁵ Fibroblasten pro cm³ Biotinte und noch bevorzugter zwischen 1 und 5 x 10⁵ Fibroblasten pro cm³ Biotinte liegt.

8. Verfahren zur Herstellung eines Ersatzes für Körpergewebe gemäß einem der Ansprüche 5 bis 7, bei dem die Menge der abgelagerten Keratinozyten zwischen 0,05 und 50 x 10⁵, vorzugsweise zwischen 0,2 und 20 x 10⁵ und noch bevorzugter zwischen 0,5 und 10 x 10⁵ Keratinozyten pro cm² der oberen flachen Oberfläche liegt.

9. Verfahren zur Herstellung eines Ersatzes für Körpergewebe gemäß einem der Ansprüche 5 bis 8, wobei der Ersatz für Körpergewebe ein Hautersatz ist, und wobei in diesem Verfahren:
(i) In einem ersten Schritt in mehreren Schichten mit einer wie in Anspruch 1 definierten Biotinte eine Hypodermis gedruckt wird, umfassend Präadipozyten und/oder reife Adipozyten,
(ii) In einem zweiten Schritt in mehreren Schichten mit einer wie in Anspruch 1 definierten Biotinte eine Dermis gedruckt wird, umfassend Fibroblasten,
(iii) In einem dritten Schritt in einer oder mehreren Schichten mit einer wie in Anspruch 1 definierten Biotinte eine Epidermis gedruckt wird, umfassend Keratinozyten.

## Claims

1. Method of fabricating a body tissue substitute, in which:
(i) a bio-ink is provided;
(ii) an aqueous solution, called "polymerization solution", is provided comprising between 1% and 5% by mass of calcium ions and between 2 U/mL and 40 U/mL, and preferably between 5 U/mL and 40 U/mL, and even more preferably between 10 U/mL and 30 U/mL) of thrombin;
(iii) said bio-ink is brought to a temperature Tl above its gel point and it is deposited on a substrate at a temperature T2 below the gel point of said bio-ink, at which it gels to form a controlled three-dimensional object called the "untreated printed object"; knowing that preferably T1 is between 27°C and 32°C and T2 is between 4°C and 20°C
(iv) said untreated printed object is treated with said polymerization solution to consolidate said untreated printed object into a body tissue substitute;
(v) said body substitute precursor is incubated in a cell culture medium,
wherein the bio-ink provided at step (i) is obtained by a fabricating method in which:
(a) a first aqueous solution is provided comprising between 5% and 40% by mass of gelatin, preferably between 6% and 30% by mass, and between 0% and 5% by mass of NaCl;
(b) a second aqueous solution is provided comprising between 1% and 12% by mass of alginate, preferably between 1% and 8% by mass, and between 0% and 5% by mass of NaCl;
(c) a third aqueous solution is provided comprising between 1% and 15% by mass of fibrinogen, preferably between 3% and 15% by mass, and living cells in suspension;
(d) a mixture is created comprising:
about 35% to 65% by volume of the first solution;
about 15% to 35% by volume of the second solution;
about 15% to 35% by volume of the third solution;
these proportions being chosen so as to add up to 100%,
and in which fabricating method:
- the order of steps (a),(b) and (c) is indifferent,
- the NaCl content is chosen such that in said first solution and said second solution combined, it is between 0.2% and 5% by mass, preferably between 0.2 and 3% by mass, and even more preferably between 0.4% and 2% by mass,
- said living cells are primary cells isolated from all human or animal tissue or organ, or animal stem cells or non-embryonic human stem cells.

2. The method of fabricating a body tissue substitute according to claim 1, **characterized in that** said living cells of the bio-ink provided at step (i) are primary cells isolated from connective or support tissue, a muscle tissue, a nerve tissue or an epithelia.

3. The method of fabricating a body tissue substitute according to claim 1, **characterized in that** said living cells of the bio-ink provided at step (i) are selected from the group composed of umbilical cord blood cells, non-embryonic human stem cells, adult stem cells, cancerous stem cells, progenitor cells, induced pluripotent stem cells.

4. The method of fabricating a body tissue substitute according to any one of claims 1 to 3, in which said untreated printed object is treated with said polymerization solution by immersion, at a temperature T3 higher than Tl and preferably between 35°C and 38°C.

5. The method of fabricating a body tissue substitute according to any one of claims 1 to 4, in which
o said body tissue precursor is a skin substitute precursor, and
o said bio-ink is a suspension comprising living fibroblast cells;
o said incubation is done at a temperature between 36°C and 38°C and it comprises a first incubation phase lasting between eight and forty days, and a second incubation phase lasting between five and ten days, knowing that an aqueous suspension of keratinocytes is deposited on the surface of said skin substitute between the first and the second phases.

6. The method of fabricating a body tissue substitute according to claim 5, in which said untreated printed object comprises an approximately flat upper surface and has a uniform distribution of fibroblasts, which is comprised between 0.2 and 10 x 10⁵, preferably between 0.2 and 2 x 10⁵fibroblasts per cm² of flat upper surface.

7. The method of fabricating a body tissue substitute according to claim 5 or 6, in which the concentration of fibroblasts is comprised between 0.6 et 12 x 10⁵ fibroblasts per cm³ of bio-ink, preferably between 1 and 7 x 10⁵ fibroblasts per cm³ of bio-ink, and even more preferably between 1 and 5 x 10⁵ fibroblasts par cm³ of bio-ink.

8. The method of fabricating a body tissue substitute according to any one of claims 5 to 7, in which the quantity of deposited keratinocytes is comprised between 0.05 and 50 x 10⁵, preferably between 0.2 and 20 x 10⁵, and more preferably between 0.5 and 10 x 10⁵, keratinocytes per cm² of flat upper surface.

9. The method of fabricating a body tissue substitute according to any one of claims 5 to 8, in which said body tissue substitute is a skin substitute, and in which method:
(i) in a first step, a hypodermis is printed in several layers with a bio-ink as defined in claim 1 comprising pre-adipocytes and/or mature adipocytes,
(ii) in a second step, a dermis is printed in several layers with a bio-ink as defined in claim 1 comprising fibroblasts,
(iii) in a third step, an epidermis is printed in one or several layers with a bio-ink as defined in claim 1 comprising keratinocytes.
